# EUROPEAN PATENT APPLICATION

(11) **EP 4 563 704 A1**
(43) Date of publication of application: **04.06.2025**
(21) Application number: 24167465.4
(22) Date of filing: 28.03.2024
(51) Int. Cl.: C12N 15/86

(54) **GENE DELIVERY VECTOR FOR DELIVERING HUMAN VEGF RECEPTOR FUSION PROTEIN AND USE THEREOF**

(30) Priority: 29.11.2023 WO PCT/CN2023/134932
(71) Applicant: Shanghai Correge Pharmaceutical Technology Co., Ltd., Shanghai 201210 (CN)
(72) Inventor: CHEN, Lihui, Shanghai, 201210 (CN); LI, Jun, Shanghai, 201210 (CN)
(74) Representative: Prüfer & Partner mbB Patentanwälte · Rechtsanwälte

(57) **Abstract**

Disclosed is a gene delivery vector, particularly a recombinant adeno-associated virus vector, for delivering an engineered human VEGF receptor fusion protein, and use thereof in the treatment of a VEGF-induced disease. Further provided is the engineered human VEGF receptor fusion protein and a nucleic acid encoding same, a vector thereof, a cell thereof, and an expression cassette thereof.

## Description

### TECHNICAL FIELD

The present invention relates to a gene delivery vector, particularly a recombinant adeno-associated virus, for delivering a human VEGF receptor fusion protein. The present invention further relates to the human VEGF receptor fusion protein, a nucleic acid encoding the fusion protein, a vector comprising the nucleic acid, and a host cell comprising the vector. The present invention further relates to an expression cassette expressing the human VEGF receptor fusion protein and to a recombinant plasmid for forming the recombinant adeno-associated virus. The present invention further relates to use of the gene delivery vector, the fusion protein, the nucleic acid, the vector, the host cell, the expression cassette, and the recombinant plasmid described above in the treatment of a VEGF-induced disease, particularly an ophthalmic disease, and a method for treating the disease using same.

### BACKGROUND

VEGF (vascular endothelial growth factor) plays a major role in the pathogenesis of a variety of eye diseases. Upon onset, there is an increase in the concentration of VEGF within the eye, and abnormal new blood vessels that easily bleed are produced. This results in severe complications such as massive hemorrhage, fibroplasia, tractional retinal detachment, and neovascular glaucoma and may also cause significant vascular leakage leading to persistent severe edema in tissues. Such ophthalmic diseases include exudative (wet) age-related macular degeneration (AMD), diabetic retinopathy (DR) combined with macular edema (DME), retinal vein occlusion (RVO) combined with macular edema (ME), central exudative chorioretinopathy, polypoidal choroidal vasculopathy (PCV), choroidal neovascularization secondary to high myopia, and macular choroidal neovascularization (CNV) secondary to other diseases. Among them, wAMD and DME have relatively higher incidences and hence are more common.

Based on the mechanism of neutralizing VEGF, a variety of antibody drugs for inhibiting ocular angiogenesis, such as Ranibizumab, Bevacizumab, Aflibercept, and Conbercept, are commercially available at present. Aflibercept is a soluble, fully humanized fusion protein consisting of Ig domain 2 of human VEGFR1, Ig domain 3 of human VEGFR2, and a hinge-Fc region of human IgG1. It can be used as a bait receptor that binds to VEGF-A, VEGF-B, and placental growth factor (PIGF), preventing them from binding to receptors and blocking their downstream biological effects. At present, the drug has been widely applied to clinical treatment of ophthalmic diseases, and the FDA has approved the clinical use of aflibercept for a number of indications, including wetAMD, RVO, DME, among others.

These drugs have been successful in treating angiogenesis-related ophthalmic diseases. However, the existing ophthalmic anti-VEGF therapies are also deficient. In general, anti-VEGF drug therapies are currently first-line treatment regimens suitable for wAMD and DME. A number of anti-VEGF drugs, such as Ranibizumab, Conbercept, and Aflibercept, have been widely approved for commercial availability. At present, all these first-line anti-VEGF drugs are antibody-based macromolecular drugs. Such drugs have short metabolic periods and half-lives and cannot act in the body for an extended period of time, necessitating frequent intravitreal injections into the patient. For example, for the treatment of neovascular AMD, the recommended administration regimen for aflibercept is monthly intravitreal injections (2 mg/injection) over the first 3 months, followed by one intravitreal injection every 8 weeks, i.e., a 3+every-8-week regimen; or monthly intravitreal injections (2 mg/injection) over the first 3 months, followed by treat-and-extend (T&E) administration, i.e., a 3+T&E regimen. The existing treatments, due to their relatively high frequency of administration, have increased the burden on patients and also imposed stricter requirements on medication adherence.

### SUMMARY

A gene encoding a drug protein (such as an antibody drug or fusion protein drug) is delivered to a diseased site in a targeting manner by means of a gene delivery vector (such as an adeno-associated virus, adenovirus, lentivirus, or lipid nanoparticle), so that the drug protein can be directly and efficiently expressed *in vivo* for a long time and stably act in the patient for a long time, reducing the frequency of administration. Theoretically, it is even possible to achieve a lasting effect with a single dose, thereby lightening the burden on patients practically.

In the present invention, the human IgG1-Fc fragment in the aflibercept protein molecule is engineered by YTE (M252Y/S254T/T256E) site-directed mutagenesis to obtain an engineered aflibercept protein drug molecule (briefly, "Fc-YTE-aflibercept"). By encoding the Fc-YTE-aflibercept protein molecule gene through a gene delivery vector, a gene therapy is obtained (briefly, rAAV-Fc-YTE" or "rAAV-Fc-YTE-aflibercept"). The inventors surprisingly found that the gene therapy drug can significantly improve the therapeutic effect on diseases of interest without changing the expression level and expression distribution of the drug molecule in the target tissue.

Thus, in one aspect, the present invention provides a gene delivery vector, comprising a nucleic acid, wherein the nucleic acid comprises an expression cassette that expresses a human VEGF receptor fusion protein; the human VEGF receptor fusion protein comprises an immunoglobulin-like domain 2 of human VEGFR1, an immunoglobulin-like domain 3 of human VEGFR2, and a hinge-Fc domain of a human immunoglobulin; the hinge-Fc domain of the human immunoglobulin comprises, according to EU numbering, substitutions M252Y, S254T, and T256E.

In some embodiments, the human VEGF receptor fusion protein further comprises an immunoglobulin-like domain 4 of human VEGFR2.

In some embodiments, the hinge-Fc domain of the human immunoglobulin is a hinge-Fc domain of human IgG1.

In some embodiments, the immunoglobulin-like domain 2 of human VEGFR1 comprises or consists of the amino acid sequence set forth in SEQ ID NO. 1 or 6, or an amino acid sequence having at least 80% sequence identity to the amino acid sequence set forth in SEQ ID NO. 1 or 6.

In some embodiments, the immunoglobulin-like domain 3 of human VEGFR2 comprises or consists of the amino acid sequence set forth in SEQ ID NO. 2, or an amino acid sequence having at least 80% sequence identity to the amino acid sequence set forth in SEQ ID NO. 2.

In some embodiments, the immunoglobulin-like domain 4 of human VEGFR2 comprises or consists of the amino acid sequence set forth in SEQ ID NO. 7, or an amino acid sequence having at least 80% sequence identity to the amino acid sequence set forth in SEQ ID NO. 7.

In some embodiments, the hinge-Fc domain of the human immunoglobulin comprises or consists of the amino acid sequence set forth in SEQ ID NO. 3 or 8, or an amino acid sequence having at least 80% sequence identity to the amino acid sequence set forth in SEQ ID NO. 3 or 8.

In some embodiments, the human VEGF receptor fusion protein comprises or consists of the amino acid sequence set forth in SEQ ID NO. 5 or 9, or an amino acid sequence having at least 80% sequence identity to the amino acid sequence set forth in SEQ ID NO. 5 or 9.

In some embodiments, the expression cassette comprises: the nucleotide sequence set forth in SEQ ID NO. 10 or 11, or a degenerate sequence of any of SEQ ID NOs. 10 and 11.

In some embodiments, the expression cassette further comprises a promoter that is upstream of a nucleotide sequence encoding the human VEGF receptor fusion protein and is operably linked thereto; the promoter is preferably selected from a cytomegalovirus (CMV) promoter, a Rous sarcoma virus (RSV) promoter, an MMT promoter, an EF-1α promoter, a U6 promoter, a chicken β-actin promoter, a CAG promoter, a CBA promoter, an RPE65 promoter, a VMD2 promoter, an RPGR promoter, an IRBP promoter, an hGRK1 promoter, a CAR promoter, an RHO promoter, a Grm6 promoter, a GRK1 promoter, and a GFAP promoter.

In some embodiments, the expression cassette further comprises a polyadenylation signal that is downstream of the nucleotide sequence encoding the human VEGF receptor fusion protein and is operably linked thereto; the polyadenylation signal is preferably selected from an SV40 early poly A, an SV40 late polyA, a rabbit globin polyA, a bGH polyA, and an HSV TK polyA.

In some embodiments, the gene delivery vector is (a) a viral vector, preferably a lentivirus, retrovirus, adenovirus, adeno-associated virus, herpesvirus, poxvirus, papovavirus, baculovirus, or papillomavirus, and more preferably an adeno-associated virus or lentivirus; or (b) a non-viral vector, preferably a plasmid, liposome, nanoparticle, polymer, transposon, exosome, or bacterial vector.

In some embodiments, the vector is an adeno-associated virus vector, comprising: (a) a recombinant adeno-associated virus capsid, and (b) the nucleic acid packaged within the recombinant adeno-associated virus capsid.

In some embodiments, the recombinant adeno-associated virus capsid comprises capsid proteins of an adeno-associated virus selected from the following serotypes: AAV1, AAV2, AAV3, AAV4, AAV5, AAV6, AAV7, AAV8, AAV9, AAV10, AAV11, AAV12, and hybrids thereof, and capsid protein mutants obtained by engineering with the above capsid proteins as backbones; preferably, the recombinant adeno-associated virus capsid comprises capsid proteins of an adeno-associated virus selected from AAV1, AAV2, AAV5, AAV7, AAV8, and hybrids thereof.

In some embodiments, the genome of the adeno-associated virus vector is single-stranded or double-stranded and is preferably an scAAV, ssAAV, or cceAAV.

In some embodiments, provided is a recombinant adeno-associated virus, comprising: (a) an rAAV capsid; and (b) a nucleic acid packaged within the rAAV capsid, the nucleic acid comprising in 5' to 3' order: (i) a 5' AAV ITR; (ii) a promoter; (iii) a polynucleotide encoding the human VEGF receptor fusion protein provided by the present invention; (iv) a polyA; and (v) a 3' AAV ITR.

In some embodiments, the rAAV capsid comprises capsid proteins of AAV1, AAV2, AAV5, AAV7, or AAV8, preferably capsid proteins of AAV8.

In some embodiments, the promoter is a cytomegalovirus (CMV) promoter, EF-1α promoter, chicken β-actin promoter, GRK1 promoter, or CAG promoter, preferably a CMV promoter.

In some embodiments, the polyA is an SV40 late polyA or rabbit globin poly A, preferably an SV40 late polyA.

In some embodiments, the 5' AAV ITR and/or the 3' AAV ITR are/is selected from ITRs of AAV1, AAV2, AAV3, AAV4, AAV5, and AAV6.

In some embodiments, the present invention provides an expression cassette, comprising in 5' to 3' order: (i) a promoter; (ii) a polynucleotide encoding the human VEGF receptor fusion protein provided by the present invention; and (iii) a polyA.

In some embodiments, the promoter is a cytomegalovirus (CMV) promoter, EF-1α promoter, chicken β-actin promoter, GRK1 promoter, or CAG promoter, preferably a CMV promoter.

In some embodiments, the polyA is an SV40 late polyA or rabbit globin polyA, preferably an SV40 late polyA.

In some embodiments, the expression cassette comprises a 5' AAV ITR and a 3' AAV ITR at the 5' end of the promoter and at the 3' end of the polyA, respectively.

Another aspect of the present invention provides a human VEGF receptor fusion protein, comprising an immunoglobulin-like domain 2 of human VEGFR1, an immunoglobulin-like domain 3 of human VEGFR2, and a hinge-Fc domain of a human immunoglobulin, wherein the hinge-Fc domain of the human immunoglobulin comprises, according to EU numbering, substitutions M252Y, S254T, and T256E.

In some embodiments, the human VEGF receptor fusion protein further comprises an immunoglobulin-like domain 4 of human VEGFR2; preferably, the immunoglobulin-like domain 4 of human VEGFR2 comprises or consists of the amino acid sequence set forth in SEQ ID NO. 7, or an amino acid sequence having at least 80% sequence identity to the amino acid sequence set forth in SEQ ID NO. 7.

In some embodiments, the hinge-Fc domain of the human immunoglobulin is a hinge-Fc domain of human IgG1; preferably, the hinge-Fc domain of human IgG1 comprises or consists of the amino acid sequence set forth in SEQ ID NO. 3 or 8, or an amino acid sequence having at least 80% sequence identity to the amino acid sequence set forth in SEQ ID NO. 3 or 8.

In some embodiments, the immunoglobulin-like domain 2 of human VEGFR1 comprises or consists of the amino acid sequence set forth in SEQ ID NO. 1 or 6, or an amino acid sequence having at least 80% sequence identity to the amino acid sequence set forth in SEQ ID NO. 1 or 6.

In some embodiments, the immunoglobulin-like domain 3 of human VEGFR2 comprises or consists of the amino acid sequence set forth in SEQ ID NO. 2, or an amino acid sequence having at least 80% sequence identity to the amino acid sequence set forth in SEQ ID NO. 2.

In some embodiments, the human VEGF receptor fusion protein comprises or consists of the amino acid sequence set forth in SEQ ID NO. 5 or 9, or an amino acid sequence having at least 80% sequence identity to the amino acid sequence set forth in SEQ ID NO. 5 or 9.

In some embodiments, the present invention provides a polynucleotide encoding any of the human VEGF receptor fusion proteins. Preferably, the polynucleotide comprises or consists of the nucleotide sequence set forth in SEQ ID NO. 10 or 11, or a degenerate sequence of any of SEQ ID NOs. 10 and 11.

In some embodiments, the present invention provides a vector comprising the polynucleotide, wherein preferably, the vector is a plasmid; preferably, the vector is constructed for formation of an ssAAV, scAAV, or cceAAV.

In some embodiments, the present invention provides a host cell comprising the vector, wherein preferably, the host cell is a HEK293 cell or sf9 cell.

Other aspects and advantages of the present invention are apparent from the following detailed description and examples.

### BRIEF DESCRIPTION OF THE DRAWINGS

FIG. 1 shows a plasmid map of a master plasmid used to form the rAAV-Fc-YTE-aflibercept of the present invention, according to one example of the present invention.
FIG. 2 shows a schematic diagram of the structure of the rAAV-Fc-YTE-aflibercept prepared according to one example of the present invention.
FIG. 3 shows FFA spot scores; **p < 0.05 and* ***p < 0.01* indicate that there are significant differences between the rAAV-Eylea, rAAV-Fc-YTE, and positive drug molecule groups and the negative control reagent group.
FIG. 4 shows FFA spot areas; **p < 0.05 and **p < 0.01* indicate that there are significant differences between the rAAV-Fc-YTE group and the rAAV-Eylea and positive drug molecule groups; there is a significant difference between the negative control reagent group and the positive drug molecule group; and there is no significant difference between the rAAV-Eylea group and the positive drug molecule group.
FIG. 5 shows the expression distribution level of the drug protein molecule of each group as measured by Elisa.
FIG. 6 shows the expression distributions of the genes of the drug proteins of interest across various layers of retinal cells as measured by BaseScope technology.
FIG. 7 shows a comparison of FFA spot scores following intraocular IVT administration of protein drugs.
FIG. 8 shows a comparison of FFA spot areas following intraocular IVT administration of protein drugs.

### DETAILED DESCRIPTION

### Definitions

The term "viral vector" or "viral particle" as used interchangeably herein refers to a viral particle consisting of at least one enveloped or non-enveloped viral capsid protein and a packaged recombinant viral genome. The viral particle comprises a recombinant viral genome having a heterologous polynucleotide, and the heterologous polynucleotide comprises a nucleotide sequence encoding the human VEGF receptor fusion protein of the present invention and an optional transcriptional regulatory region.

The terms "adeno-associated virus vector", "AAV vector", "adeno-associated virus", "AAV virus", "AAV viral particle", "AAV viral particle", and "AAV particle" as used interchangeably herein refer to a viral particle consisting of at least one AAV capsid protein (preferably consisting of all the capsid proteins of a particular AAV serotype) and a packaged recombinant viral genome. The particle comprises a recombinant viral genome; the recombinant viral genome has a heterologous polynucleotide (encoding the human VEGF receptor fusion protein of the present invention) and a transcriptional regulatory region; the transcriptional regulatory region comprises at least a promoter. The transcriptional regulatory region may also comprise a polyA.

The term "recombinant host cell" (or briefly "host cell") as used herein refers to a cell into which an exogenous nucleic acid and/or a recombinant vector have/has been introduced. It should be understood that "recombinant host cell" and "host cell" refer not only to a particular subject cell but to the progeny of such a cell. Due to mutation or environmental influence, certain modifications may occur in the progeny. Thus, such progeny may differ from the parent cell in practice, but they are still included within the scope of the term "host cell" as used herein.

The term "recombinant viral genome" refers to a viral genome or a portion thereof into which at least one expression cassette is inserted. The term "AAV recombinant viral genome" as used herein refers to an AAV genome into which at least one expression cassette polynucleotide is inserted. The smallest "genome" of the AAV genome according to the present invention generally includes cis-acting 5' and 3' inverted terminal repeats (ITRs) and an expression cassette.

The term "expression cassette" as used herein refers to a nucleic acid construct generated recombinantly or synthetically with a series of specified nucleic acid elements that permit transcription of a particular nucleic acid in a target cell. The expression cassette of the AAV recombinant viral genome of the AAV vector according to the present invention may comprise a transcriptional regulatory region operably linked to a nucleotide sequence encoding the human VEGF receptor fusion protein of the present invention.

The term "transcriptional regulatory region" as used herein refers to a nucleic acid fragment capable of regulating the expression of one or more genes. The transcriptional regulatory region according to the present invention comprises a promoter and an optional enhancer. The term "promoter" as used herein refers to a nucleic acid fragment located upstream of a polynucleotide sequence, which functions to control the transcription of one or more polynucleotides. Any kind of promoter may be used in the present invention, including inducible promoters, constitutive promoters, and tissue-specific promoters. The term "inducible promoter" as used herein refers to a promoter that is regulated physiologically or developmentally, for example, by application of a chemical inducer. For example, it may be a tetracycline-inducible promoter, a mifepristone (RU-486)-inducible promoter, or the like. The term "constitutive promoter" as used herein refers to a promoter whose activity remains relatively constant across all cells of an organism or through most developmental stages, with little or no consideration for the cellular environmental conditions. Examples of constitutive promoters include, but are not limited to, a retroviral Rous sarcoma virus (RSV) LTR promoter (optionally with an RSV enhancer), a cytomegalovirus (CMV) promoter (optionally with a CMV enhancer), an SV40 promoter, a dihydrofolate reductase promoter, a β-actin promoter, a phosphoglycerate kinase (PGK) promoter, and an EF1a promoter. Exemplary viral promoters that function constitutively in cells include, for example, an SV40 early promoter region, a promoter contained in the 3' long terminal repeat of the Rous sarcoma virus, or a herpesvirus thymidine kinase promoter.

The term "enhancer" as used herein refers to a DNA sequence element that binds to a transcription factor to increase transcription of a gene. Examples of enhancers may be, but are not limited to, an RSV enhancer, a CMV enhancer, an HCR enhancer, and the like.

The term "operably linked" as used herein refers to the functional relation and location of a promoter sequence with respect to a polynucleotide of interest (for example, a promoter or enhancer is operably linked to a coding sequence if it affects the transcription of the sequence). Generally, a promoter operably linked is contiguous to the sequence of interest. However, an enhancer does not have to be contiguous to the sequence of interest to control its expression. In another embodiment, the promoter is contiguous to a nucleotide sequence encoding the human VEGF receptor fusion protein of the present invention.

The term "therapeutically effective amount" refers to an amount of the viral vector encoding the human VEGF receptor fusion protein of the present invention that is non-toxic but sufficient to provide a desired biological result. The result can be reduction and/or alleviation of the signs, symptoms, or causes of a disease, or any other desired alteration of a biological system. For example, the therapeutically effective amount of the AAV vector according to the present invention is an amount sufficient to produce a desired biological result.

The term "Cap protein" as used herein refers to a polypeptide having at least one functional activity of a native AAV Cap protein (e.g., VP1, VP2, or VP3). Examples of functional activities of Cap proteins include the ability to induce capsid formation, promote accumulation of a single-stranded DNA, promote packaging of an AAV DNA into a capsid, bind a cellular receptor, and promote entry of a viral particle into a host cell. In principle, any Cap protein can be used in the context of the present invention. The term "capsid" as used herein refers to the packaging structure of a viral genome. A capsid consists of several oligomeric structural subunits made of proteins. For example, an AAV has an icosahedral capsid formed by the interaction of three capsid proteins VP1, VP2, and VP3.

The term "Rep protein" as used herein refers to a polypeptide having at least one functional activity of a native AAV rep protein (e.g., Rep 40, 52, 68, or 78). A "functional activity" of a Rep protein is any activity associated with the physiological function of the protein. Other functions include regulation of transcription from AAV (or other heterologous) promoters and site-specific integration of an AAV DNA into a host chromosome. In a particular embodiment, the AAV rep gene is derived from the serotypes AAV1, AAV2, AAV4, AAV5, AAV6, AAV7, AAV8, AAV9, AAV10, AAV11, AAV12, and hybrids thereof, and capsid protein mutants obtained by engineering with the above capsid proteins as backbones.

"Viral proteins upon which AAV replication is dependent" as used herein refers to polypeptides upon which AAV replication is dependent (i.e., "accessory function polypeptides"). Accessory functions include those functions required for AAV replication, including, but not limited to, those involved in activation of AAV gene transcription, stage-specific AAV mRNA splicing, AAV DNA replication, synthesis of cap expression products, and AAV capsid assembly. Virus-based accessory functions are derived from any of the known helper viruses, such as adenoviruses, herpesviruses (other than herpes simplex virus type 1), and the vaccinia virus. Helper functions include, but are not limited to, adenoviruses E1, E2a, VA, and E4 or herpesviruses UL5, ULB, UL52, and UL29, and herpesvirus polymerase. In another embodiment, the viral proteins upon which AAV replication is dependent are derived from adenoviruses.

The term "adeno-associated virus ITRs" or "AAV ITRs" as used herein refers to inverted terminal repeats present at both ends of a genomic DNA strand of an adeno-associated virus. ITR sequences are essential for effective proliferation of the AAV genome. Another property of these sequences is their ability to form a hairpin. This property contributes to their self-priming, thereby allowing independent synthesis of the second DNA strand. Procedures for modifying these ITR sequences are known in the art.

The term "polyadenylation signal" or "polyA" as used herein relates to a nucleic acid sequence that mediates ligation of a polyadenosine strand to the 3' end of an mRNA. Suitable polyA signals include, but are not limited to, an SV40 early polyA signal, an SV40 late polyA signal, a rabbit globin polyA, a bGH polyA, and an HSV thymidine kinase (TK) polyA signal.

The term "nucleotide or nucleic acid sequence" is used interchangeably herein with "polynucleotide" and refers to any polymeric form of nucleotides of any length.

The term "signal peptide" as used herein refers to a sequence of amino acid residues (10-30 residues in length) that binds to the amino-terminus of a nascent protein of interest during translation of a protein. Signal peptides are recognized by the signal recognition particle (SRP) and, after translocation into the endoplasmic reticulum, cleaved by a signal peptidase.

The term "subject" as used herein refers to an individual mammal, such as a human, a non-human primate (e.g., chimpanzees and other apes and monkey species), a farm animal (e.g., birds, fish, cattle, sheep, pigs, goats, and horses), a domestic mammal (e.g., dogs and cats), or a laboratory animal (e.g., rodents, such as mice, rats, and guinea pigs). The term includes subjects of any age or sex. In another embodiment, the subject is a mammal, preferably a human.

As used herein, the term "Fc" refers to a human IgG (immunoglobulin) Fc domain. Subtypes of IgG, such as IgG1, IgG2, IgG3, and IgG4, can all be used as Fc domains. "Fc region" or "Fc domain" as used herein is the portion of an IgG molecule that is associated with a crystallizable fragment obtained by papain digestion of an IgG molecule. It has no antigen binding activity but contains the carbohydrate moiety and the binding sites for complement and Fc receptors (including the FcRn receptor). The Fc region contains the entire second constant domain CH2 (residues 231-340 of human IgG1, according to the EU numbering system, which is also used hereinafter) and the entire third constant domain CH3 (residues 341-447). The term "hinge" encompasses the entire hinge region that extends from the N-terminus of the Fc region (residues 216-230) or a fragment thereof (e.g., 221-230). "IgG hinge-Fc region" or "hinge-Fc domain" refers to a region of an IgG molecule that consists of the Fc region (residues 231-447) and the hinge region that extends from the N-terminus of the Fc region or a fragment thereof.

A single-stranded AAV (ssAAV) as used herein refers to an rAAV that has the coding sequence of a transgene expression cassette on a separate strand and is packaged in a viral capsid. It requires a process of transforming from a single strand to a double strand. Synthesis of the second strand of a viral DNA has been shown to be a rate-limiting step in viral gene expression.

A self-complementary AAV (scAAV) as used herein is formed by deleting a D sequence (packaging signal) from an ITR on the right side of an ssAAV genome and mutating a terminal resolution site (Δtrs), which can prevent a Rep protein from modifying and regulating an unwinding nick and enhance packaging of a self-complementary double-stranded DNA. After a double-stranded AAV virus enters a cell, expression is directly performed without the need for a process of transforming from a single strand to a double strand, and the expression level is relatively high.

A covalently closed end AAV (cceAAV) as used herein is a newly emerging rAAV system, which is formed by blocking one end of the complementary double-stranded DNA of an AAV genome through, for example, an shRNA or oligonucleotide strand. One exemplary cceAAV is the cceAAV system described in WO2020/092904, which is incorporated herein by reference in its entirety.

### Gene Delivery Vector

A first aspect of the present invention provides a gene delivery vector, comprising a nucleic acid, wherein the nucleic acid comprises an expression cassette that expresses a human VEGF receptor fusion protein; the human VEGF receptor fusion protein comprises an immunoglobulin-like domain 2 of human VEGFR1, an immunoglobulin-like domain 3 of human VEGFR2, and a hinge-Fc domain of a human immunoglobulin; the hinge-Fc domain of the human immunoglobulin comprises, according to EU numbering, substitutions M252Y, S254T, and T256E.

In some embodiments, the human VEGF receptor fusion protein further comprises an immunoglobulin-like domain 4 of human VEGFR2. Thus, in these embodiments, the present invention provides a gene delivery vector, comprising a nucleic acid, wherein the nucleic acid comprises an expression cassette that expresses a human VEGF receptor fusion protein; the human VEGF receptor fusion protein comprises an immunoglobulin-like domain 2 of human VEGFR1, an immunoglobulin-like domain 3 of human VEGFR2, an immunoglobulin-like domain 4 of human VEGFR2, and a hinge-Fc domain of a human immunoglobulin; the hinge-Fc domain of the human immunoglobulin comprises, according to EU numbering, substitutions M252Y, S254T, and T256E.

In some embodiments, the hinge-Fc domain of the human immunoglobulin is a hinge-Fc domain of human IgG1. Thus, in these embodiments, the present invention provides a gene delivery vector, comprising a nucleic acid, wherein the nucleic acid comprises an expression cassette that expresses a human VEGF receptor fusion protein; the human VEGF receptor fusion protein comprises an immunoglobulin-like domain 2 of human VEGFR1, an immunoglobulin-like domain 3 of human VEGFR2, and a hinge-Fc domain of human IgG1; the hinge-Fc domain of human IgG1 comprises, according to EU numbering, substitutions M252Y, S254T, and T256E. In some embodiments, the present invention provides a gene delivery vector, comprising a nucleic acid, wherein the nucleic acid comprises an expression cassette that expresses a human VEGF receptor fusion protein; the human VEGF receptor fusion protein comprises an immunoglobulin-like domain 2 of human VEGFR1, an immunoglobulin-like domain 3 of human VEGFR2, an immunoglobulin-like domain 4 of human VEGFR2, and a hinge-Fc domain of human IgG1; the hinge-Fc domain of human IgG1 comprises, according to EU numbering, substitutions M252Y, S254T, and T256E.

In any of the above embodiments, preferably, in the expression cassette that expresses the human VEGF receptor fusion protein, nucleic acids encoding the immunoglobulin-like domain 2 of human VEGFR1, the immunoglobulin-like domain 3 of human VEGFR2, the immunoglobulin-like domain 4 of human VEGFR2, and the hinge-Fc domain of the human immunoglobulin are sequentially arranged in the direction from 5' to 3'. In other embodiments, in the expression cassette that expresses the human VEGF receptor fusion protein, the nucleic acids encoding the immunoglobulin-like domain 2 of human VEGFR1, the immunoglobulin-like domain 3 of human VEGFR2, and the immunoglobulin-like domain 4 of human VEGFR2 may be arranged in any order in the direction from 5' to 3', with the nucleic acid encoding the hinge-Fc domain of the human immunoglobulin at their 3' end. In other embodiments, in the expression cassette that expresses the human VEGF receptor fusion protein, the nucleic acids encoding the immunoglobulin-like domain 2 of human VEGFR1 and the immunoglobulin-like domain 3 of human VEGFR2 may be arranged in any order in the direction from 5' to 3', with the nucleic acid encoding the hinge-Fc domain of the human immunoglobulin at their 3' end.

In any of the above embodiments, preferably, the domains are directly linked to each other. In other embodiments, the domains may be linked to each other by peptide linkers. Suitable peptide linkers are known in the art and are typically composed of multiple glycines and serines. The present invention contemplates that any suitable peptide linker may be used to link the domains of the human VEGF receptor fusion protein.

In any of the above embodiments, the hinge-Fc domain of human IgG1 may comprise, according to EU numbering, one or more of the substitutions M428L and N434S in addition to the substitutions M252Y, S254T, and T256E; they are known to have the effect of enhancing the interaction with the FcRn receptor. For example, in some embodiments, the hinge-Fc domain of human IgG1 comprises, according to EU numbering, substitutions M252Y, S254T, and T256E, as well as the substitution M428L. In some other embodiments, the hinge-Fc domain of human IgG1 comprises, according to EU numbering, substitutions M252Y, S254T, and T256E, as well as the substitution N434S. In some other embodiments, the hinge-Fc domain of human IgG1 comprises, according to EU numbering, substitutions M252Y, S254T, and T256E, as well as the substitutions M428L and N434S. The present invention contemplates that the hinge-Fc domain of human IgG1 may comprise more substitutions.

In any of the above embodiments, the immunoglobulin-like domain 2 of human VEGFR1 may comprise or be: the amino acid sequence set forth in SEQ ID NO. 1 or 6, or an amino acid sequence having at least 80% sequence identity to the amino acid sequence set forth in SEQ ID NO. 1 or 6. Thus, in some embodiments, the immunoglobulin-like domain 2 of human VEGFR1 comprises or consists of the amino acid sequence set forth in SEQ ID NO. 1 or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 1. In some embodiments, the immunoglobulin-like domain 2 of human VEGFR1 comprises or consists of the amino acid sequence set forth in SEQ ID NO. 6 or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 6. In some embodiments, the immunoglobulin-like domain 2 of human VEGFR1 comprises or consists of an amino acid sequence obtained by adding or removing 1 to 10, 1 to 5, or 1 to 3 amino acid residues to or from the N-terminus and/or C-terminus of the amino acid sequence set forth in SEQ ID NO. 1 or 6. For example, in some embodiments, the immunoglobulin-like domain 2 of human VEGFR1 comprises or consists of an amino acid sequence obtained by adding 1 to 3 amino acid residues to the N-terminus of the amino acid sequence set forth in SEQ ID NO. 1, wherein the amino acid residues added may be the amino acid residues at the corresponding positions of immunoglobulin-like domain 2 of wild-type human VEGFR1. For example, in some embodiments, the immunoglobulin-like domain 2 of human VEGFR1 comprises or consists of an amino acid sequence obtained by adding 1 to 3 amino acid residues to the C-terminus of the amino acid sequence set forth in SEQ ID NO. 6, wherein the amino acid residues added may be the amino acid residues at the corresponding positions of immunoglobulin-like domain 2 of wild-type human VEGFR1.

In any of the above embodiments, the immunoglobulin-like domain 3 of human VEGFR2 may comprise or be: the amino acid sequence set forth in SEQ ID NO. 2, or an amino acid sequence having at least 80% sequence identity to the amino acid sequence set forth in SEQ ID NO. 2. Thus, in some embodiments, the immunoglobulin-like domain 3 of human VEGFR2 comprises or consists of the amino acid sequence set forth in SEQ ID NO. 2, or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 2. In some embodiments, the immunoglobulin-like domain 3 of human VEGFR2 comprises or consists of an amino acid sequence obtained by adding or removing 1 to 10, 1 to 5, or 1 to 3 amino acid residues to or from the N-terminus and/or C-terminus of the amino acid sequence set forth in SEQ ID NO. 2. For example, in some embodiments, the immunoglobulin-like domain 3 of human VEGFR2 comprises or consists of an amino acid sequence obtained by adding 1 to 3 amino acid residues to the N-terminus of the amino acid sequence set forth in SEQ ID NO. 2, wherein the amino acid residues added may be the amino acid residues at the corresponding positions of immunoglobulin-like domain 3 of wild-type human VEGFR2.

In any of the above embodiments, the immunoglobulin-like domain 4 of human VEGFR2 may comprise or be: the amino acid sequence set forth in SEQ ID NO. 7, or an amino acid sequence having at least 80% sequence identity to the amino acid sequence set forth in SEQ ID NO. 7. Thus, in some embodiments, the immunoglobulin-like domain 4 of human VEGFR2 comprises or consists of the amino acid sequence set forth in SEQ ID NO. 7, or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 7. In some embodiments, the immunoglobulin-like domain 4 of human VEGFR2 comprises or consists of an amino acid sequence obtained by adding or removing 1 to 10, 1 to 5, or 1 to 3 amino acid residues to or from the N-terminus and/or C-terminus of the amino acid sequence set forth in SEQ ID NO. 7. For example, in some embodiments, the immunoglobulin-like domain 4 of human VEGFR2 comprises or consists of an amino acid sequence obtained by adding 1 to 3 amino acid residues to the N-terminus of the amino acid sequence set forth in SEQ ID NO. 7, wherein the amino acid residues added may be the amino acid residues at the corresponding positions of immunoglobulin-like domain 4 of wild-type human VEGFR2.

Thus, in some embodiments, the present invention provides a gene delivery vector, comprising a nucleic acid, wherein the nucleic acid comprises an expression cassette that expresses a human VEGF receptor fusion protein; the human VEGF receptor fusion protein comprises, from N-terminus to C-terminus, an immunoglobulin-like domain 2 of human VEGFR1 whose amino acid sequence is set forth in SEQ ID NO. 1 or 6 or has at least 80% sequence identity thereto, an immunoglobulin-like domain 3 of human VEGFR2 whose amino acid sequence is set forth in SEQ ID NO. 2 or has at least 80% sequence identity thereto, and a hinge-Fc domain of a human immunoglobulin in sequence; the hinge-Fc domain of the human immunoglobulin comprises, according to EU numbering, substitutions M252Y, S254T, and T256E.

In some other embodiments, the present invention provides a gene delivery vector, comprising a nucleic acid, wherein the nucleic acid comprises an expression cassette that expresses a human VEGF receptor fusion protein; the human VEGF receptor fusion protein comprises, from N-terminus to C-terminus, an immunoglobulin-like domain 2 of human VEGFR1 whose amino acid sequence is set forth in SEQ ID NO. 1 or 6 or has at least 80% sequence identity thereto, an immunoglobulin-like domain 3 of human VEGFR2 whose amino acid sequence is set forth in SEQ ID NO. 2 or has at least 80% sequence identity thereto, an immunoglobulin-like domain 4 of human VEGFR2 whose amino acid sequence is set forth in SEQ ID NO. 7 or has at least 80% sequence identity thereto, and a hinge-Fc domain of a human immunoglobulin in sequence; the hinge-Fc domain of the human immunoglobulin comprises, according to EU numbering, substitutions M252Y, S254T, and T256E.

In any of the above embodiments, the hinge-Fc domain of the human immunoglobulin comprises or consists of the amino acid sequence set forth in SEQ ID NO. 3 or 8, or an amino acid sequence having at least 80% sequence identity to the amino acid sequence set forth in SEQ ID NO. 3 or 8, and the hinge-Fc domain of the human immunoglobulin comprises, according to EU numbering, substitutions M252Y, S254T, and T256E. Thus, in some embodiments, the hinge-Fc domain of the human immunoglobulin comprises or consists of the amino acid sequence set forth in SEQ ID NO. 3 or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 3, and the hinge-Fc domain of the human immunoglobulin comprises, according to EU numbering, substitutions M252Y, S254T, and T256E. In some embodiments, the hinge-Fc domain of the human immunoglobulin comprises or consists of the amino acid sequence set forth in SEQ ID NO. 8 or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 8, and the hinge-Fc domain of the human immunoglobulin comprises, according to EU numbering, substitutions M252Y, S254T, and T256E.

Thus, in a preferred embodiment, the present invention provides a gene delivery vector, comprising a nucleic acid, wherein the nucleic acid comprises an expression cassette that expresses a human VEGF receptor fusion protein; the human VEGF receptor fusion protein comprises, from N-terminus to C-terminus, an immunoglobulin-like domain 2 of human VEGFR1 whose amino acid sequence is set forth in SEQ ID NO. 1 or 6 or has at least 80% sequence identity thereto, an immunoglobulin-like domain 3 of human VEGFR2 whose amino acid sequence is set forth in SEQ ID NO. 2 or has at least 80% sequence identity thereto, and a hinge-Fc domain of a human immunoglobulin whose amino acid sequence is set forth in SEQ ID NO. 3 or 8 or has at least 80% sequence identity thereto in sequence; the hinge-Fc domain of the human immunoglobulin comprises, according to EU numbering, substitutions M252Y, S254T, and T256E.

In a preferred embodiment, the present invention provides a gene delivery vector, comprising a nucleic acid, wherein the nucleic acid comprises an expression cassette that expresses a human VEGF receptor fusion protein; the human VEGF receptor fusion protein comprises, from N-terminus to C-terminus, an immunoglobulin-like domain 2 of human VEGFR1 whose amino acid sequence is set forth in SEQ ID NO. 1 or 6 or has at least 80% sequence identity thereto, an immunoglobulin-like domain 3 of human VEGFR2 whose amino acid sequence is set forth in SEQ ID NO. 2 or has at least 80% sequence identity thereto, an immunoglobulin-like domain 4 of human VEGFR2 whose amino acid sequence is set forth in SEQ ID NO. 7 or has at least 80% sequence identity thereto, and a hinge-Fc domain of a human immunoglobulin whose amino acid sequence is set forth in SEQ ID NO. 3 or 8 or has at least 80% sequence identity thereto in sequence; the hinge-Fc domain of the human immunoglobulin comprises, according to EU numbering, substitutions M252Y, S254T, and T256E.

In a preferred embodiment, the present invention provides a gene delivery vector, comprising a nucleic acid, wherein the nucleic acid comprises an expression cassette that expresses a human VEGF receptor fusion protein; the human VEGF receptor fusion protein comprises, from N-terminus to C-terminus, an immunoglobulin-like domain 2 of human VEGFR1 whose amino acid sequence is set forth in SEQ ID NO. 1 or has at least 80% sequence identity thereto, an immunoglobulin-like domain 3 of human VEGFR2 whose amino acid sequence is set forth in SEQ ID NO. 2 or has at least 80% sequence identity thereto, and a hinge-Fc domain of a human immunoglobulin whose amino acid sequence is set forth in SEQ ID NO. 3 or has at least 80% sequence identity thereto in sequence; the hinge-Fc domain of the human immunoglobulin comprises, according to EU numbering, substitutions M252Y, S254T, and T256E.

In a preferred embodiment, the present invention provides a gene delivery vector, comprising a nucleic acid, wherein the nucleic acid comprises an expression cassette that expresses a human VEGF receptor fusion protein; the human VEGF receptor fusion protein comprises, from N-terminus to C-terminus, an immunoglobulin-like domain 2 of human VEGFR1 whose amino acid sequence is set forth in SEQ ID NO. 6 or has at least 80% sequence identity thereto, an immunoglobulin-like domain 3 of human VEGFR2 whose amino acid sequence is set forth in SEQ ID NO. 2 or has at least 80% sequence identity thereto, an immunoglobulin-like domain 4 of human VEGFR2 whose amino acid sequence is set forth in SEQ ID NO. 7 or has at least 80% sequence identity thereto, and a hinge-Fc domain of a human immunoglobulin whose amino acid sequence is set forth in SEQ ID NO. 8 or has at least 80% sequence identity thereto in sequence; the hinge-Fc domain of the human immunoglobulin comprises, according to EU numbering, substitutions M252Y, S254T, and T256E.

In a preferred embodiment, the human VEGF receptor fusion protein further comprises a signal peptide at the most N-terminus. One example of signal peptides comprises or consists of the amino acid sequence set forth in SEQ ID NO. 4.

In a preferred embodiment, the present invention provides a gene delivery vector, comprising a nucleic acid, wherein the nucleic acid comprises an expression cassette that expresses a human VEGF receptor fusion protein; the human VEGF receptor fusion protein comprises a hinge-Fc domain of a human immunoglobulin; the human VEGF receptor fusion protein comprises or consists of the amino acid sequence set forth in SEQ ID NO. 5, or an amino acid sequence having at least 80% sequence identity to the amino acid sequence set forth in SEQ ID NO. 5; the hinge-Fc domain of the human immunoglobulin comprises, according to EU numbering, substitutions M252Y, S254T, and T256E.

In a preferred embodiment, the present invention provides a gene delivery vector, comprising a nucleic acid, wherein the nucleic acid comprises an expression cassette that expresses a human VEGF receptor fusion protein; the human VEGF receptor fusion protein comprises a hinge-Fc domain of a human immunoglobulin; the human VEGF receptor fusion protein comprises or consists of the amino acid sequence set forth in SEQ ID NO. 9, or an amino acid sequence having at least 80% sequence identity to the amino acid sequence set forth in SEQ ID NO. 9; the hinge-Fc domain of the human immunoglobulin comprises, according to EU numbering, substitutions M252Y, S254T, and T256E.

In a preferred embodiment, the present invention provides a gene delivery vector, comprising a nucleic acid, wherein the nucleic acid comprises an expression cassette that expresses a human VEGF receptor fusion protein; the human VEGF receptor fusion protein comprises a hinge-Fc domain of a human immunoglobulin; the human VEGF receptor fusion protein is aflibercept, and the hinge-Fc domain of the human immunoglobulin comprises, according to EU numbering, substitutions M252Y, S254T, and T256E.

In a preferred embodiment, the present invention provides a gene delivery vector, comprising a nucleic acid, wherein the nucleic acid comprises an expression cassette that expresses a human VEGF receptor fusion protein; the human VEGF receptor fusion protein comprises a hinge-Fc domain of a human immunoglobulin; the human VEGF receptor fusion protein is conbercept, and the hinge-Fc domain of the human immunoglobulin comprises, according to EU numbering, substitutions M252Y, S254T, and T256E.

In a preferred embodiment, the present invention provides a gene delivery vector, comprising a nucleic acid, wherein the nucleic acid comprises an expression cassette that expresses a human VEGF receptor fusion protein; the human VEGF receptor fusion protein is encoded by the nucleotide sequence set forth in SEQ ID NO. 10 or 11 or a degenerate sequence of any of SEQ ID NOs. 10 and 11.

In a preferred embodiment, the present invention provides a gene delivery vector, comprising a nucleic acid, wherein the nucleic acid comprises an expression cassette that expresses a human VEGF receptor fusion protein; the human VEGF receptor fusion protein is encoded by the nucleotide sequence set forth in SEQ ID NO. 10 or a codon degenerate sequence thereof.

In a preferred embodiment, the present invention provides a gene delivery vector, comprising a nucleic acid, wherein the nucleic acid comprises an expression cassette that expresses a human VEGF receptor fusion protein; the human VEGF receptor fusion protein is encoded by the nucleotide sequence set forth in SEQ ID NO. 11 or a codon degenerate sequence thereof.

In the present invention, a codon degenerate sequence refers to a variant of a reference sequence (e.g., the nucleotide sequence set forth in SEQ ID NO. 10 or 11) obtained according to codon degeneracy. As is known in the art, the variant can be obtained by optimizing the reference sequence with respect to, but not limited to, one or more of codon frequency, mRNA secondary structure, GC content, RNase splice site, repeat sequence, etc. without changing the amino acid sequence translated from the reference sequence.

In any of the above embodiments, the expression cassette for the human VEGF receptor fusion protein may comprise a promoter that is upstream of the nucleotide sequence encoding the human VEGF receptor fusion protein and is operably linked thereto. In a preferred embodiment, the promoter is selected from a cytomegalovirus (CMV) promoter, a Rous sarcoma virus (RSV) promoter, an MMT promoter, an EF-1α promoter, a U6 promoter, a chicken β-actin promoter, a CAG promoter, a CBA promoter, an RPE65 promoter, a VMD2 promoter, an RPGR promoter, an IRBP promoter, an hGRK1 promoter, a CAR promoter, an RHO promoter, a Grm6 promoter, and a GFAP promoter. In a more preferred embodiment, the promoter is a CMV promoter.

In some embodiments, the promoter is a tissue-specific promoter. In some embodiments, the tissue-specific promoter is an eye-specific promoter. Examples of eye-specific promoters include a retinoschisin proximal promoter, an interphotoreceptor retinoid-binding protein enhancer (RS/IRBPa), rhodopsin kinase (RK), RPE65, and a human cone opsin promoter. In some embodiments, the promoter is a chicken β-actin (CB) promoter. The chicken β-actin promoter may be a short chicken β-actin promoter or a long chicken β-actin promoter. In some embodiments, the promoter (e.g., a chicken β-actin promoter) comprises an enhancer sequence, e.g., a cytomegalovirus (CMV) enhancer sequence. The CMV enhancer sequence may be a short CMV enhancer sequence or a long CMV enhancer sequence. In some embodiments, the promoter comprises a long CMV enhancer sequence and a long chicken β-actin promoter. In some embodiments, the promoter comprises a short CMV enhancer sequence and a short chicken β-actin promoter. However, one skilled in the art knows that a short CMV enhancer can be used with a long CB promoter and that a long CMV enhancer can be used with a short CB promoter (or vice versa).

In some embodiments, the expression cassette may further comprise one or more introns. In some embodiments, at least one intron is located between the promoter/enhancer sequence and the transgene. In some instances, a promoter or regulatory sequence element may be used to direct selective expression in ocular cells or ocular tissue. For example, promoters, sequence elements, or regulatory sequences found in a particular ocular cell type (e.g., retinal pigment epithelial cells) may be used in a suitable expression construct (e.g., an RPE65 or VMD2 promoter). In some embodiments, the intron is a synthetic or artificial (e.g., heterologous) intron. Examples of synthetic introns include an intron sequence derived from SV-40 (referred to as an SV-40T intron sequence) and an intron sequence derived from the chicken β-actin gene. In some embodiments, the transgene according to the present disclosure comprises one or more (1, 2, 3, 4, 5, or more) artificial introns. In some embodiments, one or more artificial introns are located between the promoter and the nucleic acid sequence (or referred to as the transgene) encoding the human VEGF receptor fusion protein. In some instances, an intron may refer to any sequence that is transcribable but is not translated. In some instances, an intron may refer to any sequence that is transcribed in a cell and removed from a mature RNA transcript. In some instances, an intron may comprise at least about 100, 500, 600, 700, 800, 900, 1000, 2000, 3000, 4000, or 5000 nucleotides. In some instances, an intron may be about 300 nucleotides. In some instances, an intron may be about 200 to 400 nucleotides. In some instances, a chimeric intron may be about 100 to 500 nucleotides. In some instances, an intron may be an intact naturally occurring intron or chimeric intron. In some aspects, introns may include, but are not limited to, introns described in CN104994882A, the disclosure of which is incorporated herein by reference in its entirety.

In any of the above embodiments, the expression cassette for the human VEGF receptor fusion protein may comprise a polyadenylation signal (polyA) that is downstream of the nucleotide sequence encoding the human VEGF receptor fusion protein and is operably linked thereto. In a preferred embodiment, the polyadenylation signal is selected from an SV40 early polyA, an SV40 late polyA, a rabbit globin polyA, a bGH polyA, and an HSV TK polyA. In a more preferred embodiment, the polyadenylation signal is an SV40 late polyA.

Thus, in some embodiments, the present invention provides a gene delivery vector, comprising a nucleic acid, wherein the nucleic acid comprises an expression cassette that expresses a human VEGF receptor fusion protein; the expression cassette comprises, in the direction from 5' to 3', a promoter, a nucleic acid encoding the human VEGF receptor fusion protein, and a polyA in sequence; the human VEGF receptor fusion protein comprises, from N-terminus to C-terminus, an immunoglobulin-like domain 2 of human VEGFR1, an immunoglobulin-like domain 3 of human VEGFR2, and a hinge-Fc domain of a human immunoglobulin in sequence; the hinge-Fc domain of the human immunoglobulin comprises, according to EU numbering, substitutions M252Y, S254T, and T256E.

In some embodiments, the present invention provides a gene delivery vector, comprising a nucleic acid, wherein the nucleic acid comprises an expression cassette that expresses a human VEGF receptor fusion protein; the expression cassette comprises, in the direction from 5' to 3', a promoter, a nucleic acid encoding the human VEGF receptor fusion protein, and a polyA in sequence; the human VEGF receptor fusion protein comprises, from N-terminus to C-terminus, an immunoglobulin-like domain 2 of human VEGFR1, an immunoglobulin-like domain 3 of human VEGFR2, an immunoglobulin-like domain 4 of VEGFR2, and a hinge-Fc domain of a human immunoglobulin in sequence; the hinge-Fc domain of the human immunoglobulin comprises, according to EU numbering, substitutions M252Y, S254T, and T256E.

In a preferred embodiment, the present invention provides a gene delivery vector, comprising a nucleic acid, wherein the nucleic acid comprises an expression cassette that expresses a human VEGF receptor fusion protein; the expression cassette comprises, in the direction from 5' to 3', a promoter, a nucleic acid encoding the human VEGF receptor fusion protein, and a polyA in sequence; the human VEGF receptor fusion protein comprises a hinge-Fc domain of a human immunoglobulin; the human VEGF receptor fusion protein comprises or consists of the amino acid sequence set forth in SEQ ID NO. 5, or an amino acid sequence having at least 80% sequence identity to the amino acid sequence set forth in SEQ ID NO. 5; the hinge-Fc domain of the human immunoglobulin comprises, according to EU numbering, substitutions M252Y, S254T, and T256E.

In a preferred embodiment, the present invention provides a gene delivery vector, comprising a nucleic acid, wherein the nucleic acid comprises an expression cassette that expresses a human VEGF receptor fusion protein; the expression cassette comprises, in the direction from 5' to 3', a promoter, a nucleic acid encoding the human VEGF receptor fusion protein, and a polyA in sequence; the human VEGF receptor fusion protein comprises a hinge-Fc domain of a human immunoglobulin; the human VEGF receptor fusion protein comprises or consists of the amino acid sequence set forth in SEQ ID NO. 9, or an amino acid sequence having at least 80% sequence identity to the amino acid sequence set forth in SEQ ID NO. 9; the hinge-Fc domain of the human immunoglobulin comprises, according to EU numbering, substitutions M252Y, S254T, and T256E.

In a preferred embodiment, the present invention provides a gene delivery vector, comprising a nucleic acid, wherein the nucleic acid comprises an expression cassette that expresses a human VEGF receptor fusion protein; the expression cassette comprises, in the direction from 5' to 3', a promoter, a nucleic acid encoding the human VEGF receptor fusion protein, and a polyA in sequence; the human VEGF receptor fusion protein is encoded by the nucleotide sequence set forth in SEQ ID NO. 10 or a codon degenerate sequence thereof.

In a preferred embodiment, the present invention provides a gene delivery vector, comprising a nucleic acid, wherein the nucleic acid comprises an expression cassette that expresses a human VEGF receptor fusion protein; the expression cassette comprises, in the direction from 5' to 3', a promoter, a nucleic acid encoding the human VEGF receptor fusion protein, and a polyA in sequence; the human VEGF receptor fusion protein is encoded by the nucleotide sequence set forth in SEQ ID NO. 11 or a codon degenerate sequence thereof.

A gene delivery vector that can be used in the present invention may be a viral vector or a non-viral vector. In some embodiments, the gene delivery vector is a non-viral vector. In a preferred embodiment, the non-viral vector is a plasmid, liposome, nanoparticle, polymer, transposon, exosome, or bacterial vector. In a more preferred embodiment, the non-viral vector is a plasmid, liposome, or nanoparticle. In a most preferred embodiment, the non-viral vector is a lipid nanoparticle (LNP).

In some embodiments, the gene delivery vector is a viral vector. In a preferred embodiment, the viral vector is a lentiviral, retroviral, adenoviral, adeno-associated virus, herpesvirus, poxvirus, papovavirus, baculovirus, or papillomavirus vector.

Retroviral vectors include the Moloney murine leukemia virus and HIV-based viruses. In some instances, an HIV-based viral vector may be used, wherein the HIV-based viral vector comprises at least two vectors, wherein the gag and pol genes are from the HIV genome and the env gene is from another virus. DNA viral vectors may be used. These vectors include poxvirus vectors such as orthopoxvirus or avipoxvirus vectors, and herpesvirus vectors such as herpes simplex I virus (HSV-1) vectors. HSV-1 vectors lacking one or more immediate early genes (IEs) are advantageous in that they are generally non-cytotoxic, persist in a state similar to latency in the target cell, and afford effective target cell transduction. Recombinant HSV vectors can incorporate about 30 kb of heterologous nucleic acid. Lentiviral vectors may be advantageous in that they are capable of infecting both actively dividing and non-dividing cells. They may also be highly effective in transducing human epithelial cells. Lentiviral vectors for use in the present disclosure may be derived from human and non-human (including SIV) lentiviruses. Examples of lentiviral vectors include nucleic acid sequences required for vector proliferation as well as a tissue-specific promoter operably linked to a human VEGF receptor fusion protein gene. Nucleic acid sequences may include viral LTRs, primer binding sites, polypurine tracts, att sites, and encapsidation sites. A lentiviral vector may be packaged into any suitable lentiviral capsid. A poxvirus vector may introduce a gene into the cytoplasm of a cell. An avipoxvirus vector may only result in short-term expression of a gene or nucleic acid. Adenoviral vectors, adeno-associated virus vectors, and herpes simplex virus (HSV) vectors may be used with the nucleic acid encoding the human VEGF receptor fusion protein of the present disclosure. An adenoviral vector may result in shorter-term expression (e.g., shorter than about 1 month) than an adeno-associated virus. In a more preferred embodiment, the viral vector is a lentiviral vector or an adeno-associated virus vector. In a most preferred embodiment, the viral vector is an adeno-associated virus vector.

When the gene delivery vector is an adeno-associated virus vector, the gene delivery vector is also referred to as a recombinant adeno-associated virus vector or a recombinant adeno-associated virus (rAAV). In such embodiments, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the human VEGF receptor fusion protein comprises an immunoglobulin-like domain 2 of human VEGFR1, an immunoglobulin-like domain 3 of human VEGFR2, and a hinge-Fc domain of a human immunoglobulin; the hinge-Fc domain of the human immunoglobulin comprises, according to EU numbering, substitutions M252Y, S254T, and T256E. The above description of the nucleic acid and expression cassette contained in the gene delivery vector applies to part (b) of the rAAV and will not be repeated here.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the human VEGF receptor fusion protein comprises, from N-terminus to C-terminus, an immunoglobulin-like domain 2 of human VEGFR1 whose amino acid sequence is set forth in SEQ ID NO. 1 or 6 or has at least 80% sequence identity thereto, an immunoglobulin-like domain 3 of human VEGFR2 whose amino acid sequence is set forth in SEQ ID NO. 2 or has at least 80% sequence identity thereto, and a hinge-Fc domain of a human immunoglobulin whose amino acid sequence is set forth in SEQ ID NO. 3 or 8 or has at least 80% sequence identity thereto in sequence; the hinge-Fc domain of the human immunoglobulin comprises, according to EU numbering, substitutions M252Y, S254T, and T256E.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the human VEGF receptor fusion protein comprises, from N-terminus to C-terminus, an immunoglobulin-like domain 2 of human VEGFR1 whose amino acid sequence is set forth in SEQ ID NO. 1 or 6 or has at least 80% sequence identity thereto, an immunoglobulin-like domain 3 of human VEGFR2 whose amino acid sequence is set forth in SEQ ID NO. 2 or has at least 80% sequence identity thereto, an immunoglobulin-like domain 4 of human VEGFR2 whose amino acid sequence is set forth in SEQ ID NO. 7 or has at least 80% sequence identity thereto, and a hinge-Fc domain of a human immunoglobulin whose amino acid sequence is set forth in SEQ ID NO. 3 or 8 or has at least 80% sequence identity thereto in sequence; the hinge-Fc domain of the human immunoglobulin comprises, according to EU numbering, substitutions M252Y, S254T, and T256E.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the human VEGF receptor fusion protein comprises, from N-terminus to C-terminus, an immunoglobulin-like domain 2 of human VEGFR1 whose amino acid sequence is set forth in SEQ ID NO. 1 or has at least 80% sequence identity thereto, an immunoglobulin-like domain 3 of human VEGFR2 whose amino acid sequence is set forth in SEQ ID NO. 2 or has at least 80% sequence identity thereto, and a hinge-Fc domain of a human immunoglobulin whose amino acid sequence is set forth in SEQ ID NO. 3 or has at least 80% sequence identity thereto in sequence; the hinge-Fc domain of the human immunoglobulin comprises, according to EU numbering, substitutions M252Y, S254T, and T256E.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the human VEGF receptor fusion protein comprises, from N-terminus to C-terminus, an immunoglobulin-like domain 2 of human VEGFR1 whose amino acid sequence is set forth in SEQ ID NO. 6 or has at least 80% sequence identity thereto, an immunoglobulin-like domain 3 of human VEGFR2 whose amino acid sequence is set forth in SEQ ID NO. 2 or has at least 80% sequence identity thereto, an immunoglobulin-like domain 4 of human VEGFR2 whose amino acid sequence is set forth in SEQ ID NO. 7 or has at least 80% sequence identity thereto, and a hinge-Fc domain of a human immunoglobulin whose amino acid sequence is set forth in SEQ ID NO. 8 or has at least 80% sequence identity thereto in sequence; the hinge-Fc domain of the human immunoglobulin comprises, according to EU numbering, substitutions M252Y, S254T, and T256E.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the human VEGF receptor fusion protein comprises a hinge-Fc domain of a human immunoglobulin; the human VEGF receptor fusion protein comprises or consists of the amino acid sequence set forth in SEQ ID NO. 5 or 9, or an amino acid sequence having at least 80% sequence identity to the amino acid sequence set forth in SEQ ID NO. 5 or 9; the hinge-Fc domain of the human immunoglobulin comprises, according to EU numbering, substitutions M252Y, S254T, and T256E.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the human VEGF receptor fusion protein comprises a hinge-Fc domain of a human immunoglobulin; the human VEGF receptor fusion protein is aflibercept, and the hinge-Fc domain of the human immunoglobulin comprises, according to EU numbering, substitutions M252Y, S254T, and T256E.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the human VEGF receptor fusion protein comprises a hinge-Fc domain of a human immunoglobulin; the human VEGF receptor fusion protein is conbercept, and the hinge-Fc domain of the human immunoglobulin comprises, according to EU numbering, substitutions M252Y, S254T, and T256E.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the human VEGF receptor fusion protein is encoded by the nucleotide sequence set forth in SEQ ID NO. 10 or 11 or a degenerate sequence of any of SEQ ID NOs. 10 and 11.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the expression cassette comprises, in the direction from 5' to 3', a 5' AAV ITR, a promoter, a nucleic acid encoding the human VEGF receptor fusion protein, a polyA, and a 3' AAV ITR in sequence; the human VEGF receptor fusion protein comprises, from N-terminus to C-terminus, an immunoglobulin-like domain 2 of human VEGFR1, an immunoglobulin-like domain 3 of human VEGFR2, and a hinge-Fc domain of a human immunoglobulin in sequence; the hinge-Fc domain of the human immunoglobulin comprises, according to EU numbering, substitutions M252Y, S254T, and T256E.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the expression cassette comprises, in the direction from 5' to 3', a 5' AAV ITR, a promoter, a nucleic acid encoding the human VEGF receptor fusion protein, a polyA, and a 3' AAV ITR in sequence; the human VEGF receptor fusion protein comprises, from N-terminus to C-terminus, an immunoglobulin-like domain 2 of human VEGFR1, an immunoglobulin-like domain 3 of human VEGFR2, an immunoglobulin-like domain 4 of VEGFR2, and a hinge-Fc domain of a human immunoglobulin in sequence; the hinge-Fc domain of the human immunoglobulin comprises, according to EU numbering, substitutions M252Y, S254T, and T256E.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the expression cassette comprises, in the direction from 5' to 3', a 5' AAV ITR, a promoter, a nucleic acid encoding the human VEGF receptor fusion protein, a polyA, and a 3' AAV ITR in sequence; the human VEGF receptor fusion protein comprises a hinge-Fc domain of a human immunoglobulin; the human VEGF receptor fusion protein comprises or consists of the amino acid sequence set forth in SEQ ID NO. 5 or 9, or an amino acid sequence having at least 80% sequence identity to the amino acid sequence set forth in SEQ ID NO. 5 or 9; the hinge-Fc domain of the human immunoglobulin comprises, according to EU numbering, substitutions M252Y, S254T, and T256E.

In a preferred embodiment, the present invention provides an adeno-associated virus vector, comprising: (a) a recombinant adeno-associated virus capsid, and (b) a nucleic acid packaged within the recombinant adeno-associated virus capsid, wherein the nucleic acid comprises an expression cassette that expresses a human VEGF receptor fusion protein; the expression cassette comprises, in the direction from 5' to 3', a 5' AAV ITR, a promoter, a nucleic acid encoding the human VEGF receptor fusion protein, a polyA, and a 3' AAV ITR in sequence; the human VEGF receptor fusion protein is encoded by the nucleotide sequence set forth in SEQ ID NO. 10 or 11 or a codon degenerate sequence thereof.

In any of the above embodiments of adeno-associated virus vectors, the recombinant adeno-associated virus capsid may comprise capsid proteins of an adeno-associated virus selected from the following serotypes: AAV1, AAV2, AAV3, AAV4, AAV5, AAV6, AAV7, AAV8, AAV9, AAV10, AAV11, AAV12, and hybrids thereof, and capsid protein mutants obtained by engineering with the above capsid proteins as backbones. In a preferred embodiment, the recombinant adeno-associated virus capsid may comprise or consist of capsid proteins of an adeno-associated virus selected from AAV1, AAV2, AAV5, AAV7, AAV8, and hybrids thereof. In a more preferred embodiment, the recombinant adeno-associated virus capsid may comprise or consist of capsid proteins of AAV8.

Examples of genomic sequences of different AAV serotypes can be found in the literature or public databases (e.g., GenBank). For example, GenBank accession numbers are NC_001401.2 (AAV2), NC_001829.1 (AAV4), NC/006152.1 (AAV5), AF028704.1 (AAV6), NC-006260.1 (AAV7), NC.006261.1 (AAV8), AX753250.1 (AAV9), and AX753362.1 (AAV10). In some embodiments, the adeno-associated virus vector according to the present invention comprises a capsid derived from a serotype, and the serotype is selected from the serotypes AAV2, AAV5, AAV7, AAV8, AAV9, AAV10, and AAVrh10. In another embodiment, the serotype of the AAV is AAV8. If the viral vector comprises sequences encoding the capsid proteins, it can be modified to comprise an exogenous sequence to direct the AAV to one or more particular cell types, or to increase the efficiency of targeted vector delivery to a cell, or to facilitate purification or detection of the AAV, or to reduce the host response.

In some embodiments, the AAV capsid protein has a tropism for ocular or muscle tissue. In some embodiments, the ocular tissue includes ocular neurons, retina, sclera, choroid, retina, vitreous body, macula, fovea centralis, optic disc, lens, pupil, iris, aqueous humor, cornea, conjunctiva ciliary body, or optic nerve. In some embodiments, the AAV capsid proteins target an ocular cell type (e.g., photoreceptor cells or retinal cells).

In any of the above embodiments of adeno-associated virus vectors, the genome of the adeno-associated virus vector is single-stranded or double-stranded and is preferably any of an scAAV, an ssAAV, or a cceAAV. One skilled in the art may select any of the systems as needed to form the adeno-associated virus vector of the present invention.

In some embodiments, the present invention provides a recombinant adeno-associated virus, comprising: (a) an rAAV capsid; and (b) a nucleic acid packaged within the rAAV capsid, the nucleic acid comprising in 5' to 3' order: (i) a 5' AAV ITR; (ii) a promoter; (iii) a nucleic acid encoding a human VEGF receptor fusion protein; (iv) a polyA; and (v) a 3' AAV ITR, wherein the above description of the corresponding components of the gene delivery vector applies to the promoter, the nucleic acid encoding the human VEGF receptor fusion protein, and the polyA and will not be repeated here.

In a preferred embodiment, the rAAV capsid comprises or consists of capsid proteins of AAV1, AAV2, AAV5, AAV7, or AAV8, preferably capsid proteins of AAV8.

In a preferred embodiment, the 5' AAV ITR and/or the 3' AAV ITR are/is selected from ITRs of AAV1, AAV2, AAV3, AAV4, AAV5, and AAV6.

In one specific embodiment, the present invention provides a recombinant adeno-associated virus, comprising: (a) an rAAV8 capsid; and (b) a nucleic acid packaged within the rAAV8 capsid, the nucleic acid comprising in 5' to 3' order: (i) a 5' AAV ITR; (ii) a CMV promoter; (iii) a nucleic acid encoding a human VEGF receptor fusion protein; (iv) an SV40 late polyA; and (v) a 3' AAV ITR, wherein the human VEGF receptor fusion protein comprises or consists of the amino acid sequence set forth in SEQ ID NO. 5 or 9.

In one specific embodiment, the present invention provides a recombinant adeno-associated virus, comprising: (a) an rAAV8 capsid; and (b) a nucleic acid packaged within the rAAV8 capsid, the nucleic acid comprising in 5' to 3' order: (i) a 5' AAV ITR; (ii) a CMV promoter; (iii) a nucleic acid encoding a human VEGF receptor fusion protein; (iv) a rabbit globin polyA; and (v) a 3' AAV ITR, wherein the human VEGF receptor fusion protein comprises or consists of the amino acid sequence set forth in SEQ ID NO. 5 or 9.

In one specific embodiment, the present invention provides a recombinant adeno-associated virus, comprising: (a) an rAAV2 capsid; and (b) a nucleic acid packaged within the rAAV2 capsid, the nucleic acid comprising in 5' to 3' order: (i) a 5' AAV ITR; (ii) a CMV promoter; (iii) a nucleic acid encoding a human VEGF receptor fusion protein; (iv) an SV40 late polyA; and (v) a 3' AAV ITR, wherein the human VEGF receptor fusion protein comprises or consists of the amino acid sequence set forth in SEQ ID NO. 5 or 9.

In one specific embodiment, the present invention provides a recombinant adeno-associated virus, comprising: (a) an rAAV2 capsid; and (b) a nucleic acid packaged within the rAAV2 capsid, the nucleic acid comprising in 5' to 3' order: (i) a 5' AAV ITR; (ii) a CMV promoter; (iii) a nucleic acid encoding a human VEGF receptor fusion protein; (iv) a rabbit globin polyA; and (v) a 3' AAV ITR, wherein the human VEGF receptor fusion protein comprises or consists of the amino acid sequence set forth in SEQ ID NO. 5 or 9.

In one specific embodiment, the present invention provides a recombinant adeno-associated virus, comprising: (a) an rAAV1 capsid; and (b) a nucleic acid packaged within the rAAV1 capsid, the nucleic acid comprising in 5' to 3' order: (i) a 5' AAV ITR; (ii) a chicken β-actin promoter; (iii) a nucleic acid encoding a human VEGF receptor fusion protein; (iv) a rabbit globin polyA; and (v) a 3' AAV ITR, wherein the human VEGF receptor fusion protein comprises or consists of the amino acid sequence set forth in SEQ ID NO. 5 or 9.

In one specific embodiment, the present invention provides a recombinant adeno-associated virus, comprising: (a) an rAAV1 capsid; and (b) a nucleic acid packaged within the rAAV1 capsid, the nucleic acid comprising in 5' to 3' order: (i) a 5' AAV ITR; (ii) a chicken β-actin promoter; (iii) a nucleic acid encoding a human VEGF receptor fusion protein; (iv) an SV40 late polyA; and (v) a 3' AAV ITR, wherein the human VEGF receptor fusion protein comprises or consists of the amino acid sequence set forth in SEQ ID NO. 5 or 9. In some embodiments of the present invention, the recombinant adeno-associated virus is any of an scAAV, an ssAAV, or a cceAAV.

In some embodiments, the present invention provides an expression cassette, comprising in 5' to 3' order: (i) a promoter; (ii) a nucleic acid encoding a human VEGF receptor fusion protein; and (iii) a polyA, wherein the above description of the corresponding components of the gene delivery vector applies to the promoter, the nucleic acid encoding the human VEGF receptor fusion protein, and the polyA and will not be repeated here.

In some embodiments, the present invention provides an expression cassette, comprising in 5' to 3' order: (i) a 5' AAV ITR; (ii) a promoter; (iii) a nucleic acid encoding a human VEGF receptor fusion protein; (iv) a polyA; and (v) a 3' AAV ITR, wherein the above description of the corresponding components of the gene delivery vector or recombinant adeno-associated virus applies to the ITR, the promoter, the nucleic acid encoding the human VEGF receptor fusion protein, and the polyA and will not be repeated here.

Recombinant AAVs may be produced using the triple transfection (three-plasmid transfection) method. Typically, recombinant AAVs are produced by transfecting a host cell with an AAV vector (comprising a transgene flanked by ITR elements) to be packaged into AAV particles, an AAV helper function vector, and an accessory function vector. The AAV helper function vector encodes "AAV helper function" sequences (e.g., rep and cap), which provide in trans functional proteins required for AAV replication and encapsulation. The accessory function vector encodes nucleotide sequences for non-AAV-derived viral and/or cellular functions upon which AAV is dependent for replication (e.g., "accessory functions"). Accessory functions include those functions required for AAV replication, including, but not limited to, those involved in activation of AAV gene transcription, stage-specific AAV mRNA splicing, AAV DNA replication, synthesis of cap expression products, and AAV capsid assembly. Virus-based accessory functions may be derived from any of the known helper viruses, such as adenoviruses, herpesviruses (other than herpes simplex virus type 1), and the vaccinia virus.

### Human VEGF Receptor Fusion Protein and Nucleic Acid Encoding Same

Another aspect of the present invention provides a human VEGF receptor fusion protein, comprising an immunoglobulin-like domain 2 of human VEGFR1, an immunoglobulin-like domain 3 of human VEGFR2, and a hinge-Fc domain of a human immunoglobulin, wherein the hinge-Fc domain of the human immunoglobulin comprises, according to EU numbering, substitutions M252Y, S254T, and T256E.

In some embodiments, the human VEGF receptor fusion protein further comprises an immunoglobulin-like domain 4 of human VEGFR2. Thus, in these embodiments, the present invention provides a human VEGF receptor fusion protein, comprising an immunoglobulin-like domain 2 of human VEGFR1, an immunoglobulin-like domain 3 of human VEGFR2, an immunoglobulin-like domain 4 of human VEGFR2, and a hinge-Fc domain of a human immunoglobulin, wherein the hinge-Fc domain of the human immunoglobulin comprises, according to EU numbering, substitutions M252Y, S254T, and T256E.

In some embodiments, the hinge-Fc domain of the human immunoglobulin is a hinge-Fc domain of human IgG1. Thus, in these embodiments, the present invention provides a human VEGF receptor fusion protein, comprising an immunoglobulin-like domain 2 of human VEGFR1, an immunoglobulin-like domain 3 of human VEGFR2, and a hinge-Fc domain of human IgG1; the hinge-Fc domain of human IgG1 comprises, according to EU numbering, substitutions M252Y, S254T, and T256E. In some embodiments, the present invention provides a human VEGF receptor fusion protein, comprising an immunoglobulin-like domain 2 of human VEGFR1, an immunoglobulin-like domain 3 of human VEGFR2, an immunoglobulin-like domain 4 of human VEGFR2, and a hinge-Fc domain of human IgG1, wherein the hinge-Fc domain of human IgG1 comprises, according to EU numbering, substitutions M252Y, S254T, and T256E.

In any of the above embodiments, preferably, the immunoglobulin-like domain 2 of human VEGFR1, the immunoglobulin-like domain 3 of human VEGFR2, the immunoglobulin-like domain 4 of human VEGFR2, and the hinge-Fc domain of the human immunoglobulin are sequentially arranged from N-terminus to C-terminus. In other embodiments, the immunoglobulin-like domain 2 of human VEGFR1, the immunoglobulin-like domain 3 of human VEGFR2, and the immunoglobulin-like domain 4 of human VEGFR2 may be arranged in any order, with the hinge-Fc domain of the human immunoglobulin at the most C-terminus. In other embodiments, the immunoglobulin-like domain 2 of human VEGFR1 and the immunoglobulin-like domain 3 of human VEGFR2 may be arranged from N-terminus to C-terminus, with the hinge-Fc domain of the human immunoglobulin at the most C-terminus.

In any of the above embodiments, preferably, the domains are directly linked to each other. In other embodiments, the domains may be linked to each other by peptide linkers. Suitable peptide linkers are known in the art and are typically composed of multiple glycines and serines. The present invention contemplates that any suitable peptide linker may be used to link the domains of the human VEGF receptor fusion protein.

In any of the above embodiments, the hinge-Fc domain of human IgG1 may comprise, according to EU numbering, one or more of the substitutions M428L and N434S in addition to the substitutions M252Y, S254T, and T256E; they are known to have the effect of enhancing the interaction with the FcRn receptor. For example, in some embodiments, the hinge-Fc domain of human IgG1 comprises, according to EU numbering, substitutions M252Y, S254T, and T256E, as well as the substitution M428L. In some other embodiments, the hinge-Fc domain of human IgG1 comprises, according to EU numbering, substitutions M252Y, S254T, and T256E, as well as the substitution N434S. In some other embodiments, the hinge-Fc domain of human IgG1 comprises, according to EU numbering, substitutions M252Y, S254T, and T256E, as well as the substitutions M428L and N434S. The present invention contemplates that the hinge-Fc domain of human IgG1 may comprise more substitutions.

In any of the above embodiments, the immunoglobulin-like domain 2 of human VEGFR1 may comprise or be: the amino acid sequence set forth in SEQ ID NO. 1 or 6, or an amino acid sequence having at least 80% sequence identity to the amino acid sequence set forth in SEQ ID NO. 1 or 6. Thus, in some embodiments, the immunoglobulin-like domain 2 of human VEGFR1 comprises or consists of the amino acid sequence set forth in SEQ ID NO. 1 or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 1. In some embodiments, the immunoglobulin-like domain 2 of human VEGFR1 comprises or consists of the amino acid sequence set forth in SEQ ID NO. 6 or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 6. In some embodiments, the immunoglobulin-like domain 2 of human VEGFR1 comprises or consists of an amino acid sequence obtained by adding or removing 1 to 10, 1 to 5, or 1 to 3 amino acid residues to or from the N-terminus and/or C-terminus of the amino acid sequence set forth in SEQ ID NO. 1 or 6. For example, in some embodiments, the immunoglobulin-like domain 2 of human VEGFR1 comprises or consists of an amino acid sequence obtained by adding 1 to 3 amino acid residues to the N-terminus of the amino acid sequence set forth in SEQ ID NO. 1, wherein the amino acid residues added may be the amino acid residues at the corresponding positions of immunoglobulin-like domain 2 of wild-type human VEGFR1. For example, in some embodiments, the immunoglobulin-like domain 2 of human VEGFR1 comprises or consists of an amino acid sequence obtained by adding 1 to 3 amino acid residues to the C-terminus of the amino acid sequence set forth in SEQ ID NO. 6, wherein the amino acid residues added may be the amino acid residues at the corresponding positions of immunoglobulin-like domain 2 of wild-type human VEGFR1.

In any of the above embodiments, the immunoglobulin-like domain 3 of human VEGFR2 may comprise or be: the amino acid sequence set forth in SEQ ID NO. 2, or an amino acid sequence having at least 80% sequence identity to the amino acid sequence set forth in SEQ ID NO. 2. Thus, in some embodiments, the immunoglobulin-like domain 3 of human VEGFR2 comprises or consists of the amino acid sequence set forth in SEQ ID NO. 2, or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 2. In some embodiments, the immunoglobulin-like domain 3 of human VEGFR2 comprises or consists of an amino acid sequence obtained by adding or removing 1 to 10, 1 to 5, or 1 to 3 amino acid residues to or from the N-terminus and/or C-terminus of the amino acid sequence set forth in SEQ ID NO. 2. For example, in some embodiments, the immunoglobulin-like domain 3 of human VEGFR2 comprises or consists of an amino acid sequence obtained by adding 1 to 3 amino acid residues to the N-terminus of the amino acid sequence set forth in SEQ ID NO. 2, wherein the amino acid residues added may be the amino acid residues at the corresponding positions of immunoglobulin-like domain 3 of wild-type human VEGFR2.

In any of the above embodiments, the immunoglobulin-like domain 4 of human VEGFR2 may comprise or be: the amino acid sequence set forth in SEQ ID NO. 7, or an amino acid sequence having at least 80% sequence identity to the amino acid sequence set forth in SEQ ID NO. 7. Thus, in some embodiments, the immunoglobulin-like domain 4 of human VEGFR2 comprises or consists of the amino acid sequence set forth in SEQ ID NO. 7, or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 7. In some embodiments, the immunoglobulin-like domain 4 of human VEGFR2 comprises or consists of an amino acid sequence obtained by adding or removing 1 to 10, 1 to 5, or 1 to 3 amino acid residues to or from the N-terminus and/or C-terminus of the amino acid sequence set forth in SEQ ID NO. 7. For example, in some embodiments, the immunoglobulin-like domain 4 of human VEGFR2 comprises or consists of an amino acid sequence obtained by adding 1 to 3 amino acid residues to the N-terminus of the amino acid sequence set forth in SEQ ID NO. 7, wherein the amino acid residues added may be the amino acid residues at the corresponding positions of immunoglobulin-like domain 4 of wild-type human VEGFR2.

Thus, in some embodiments, the present invention provides a human VEGF receptor fusion protein, comprising, from N-terminus to C-terminus, an immunoglobulin-like domain 2 of human VEGFR1 whose amino acid sequence is set forth in SEQ ID NO. 1 or 6 or has at least 80% sequence identity thereto, an immunoglobulin-like domain 3 of human VEGFR2 whose amino acid sequence is set forth in SEQ ID NO. 2 or has at least 80% sequence identity thereto, and a hinge-Fc domain of a human immunoglobulin in sequence, wherein the hinge-Fc domain of the human immunoglobulin comprises, according to EU numbering, substitutions M252Y, S254T, and T256E.

In some other embodiments, the present invention provides a human VEGF receptor fusion protein, comprising, from N-terminus to C-terminus, an immunoglobulin-like domain 2 of human VEGFR1 whose amino acid sequence is set forth in SEQ ID NO. 1 or 6 or has at least 80% sequence identity thereto, an immunoglobulin-like domain 3 of human VEGFR2 whose amino acid sequence is set forth in SEQ ID NO. 2 or has at least 80% sequence identity thereto, an immunoglobulin-like domain 4 of human VEGFR2 whose amino acid sequence is set forth in SEQ ID NO. 7 or has at least 80% sequence identity thereto, and a hinge-Fc domain of a human immunoglobulin in sequence, wherein the hinge-Fc domain of the human immunoglobulin comprises, according to EU numbering, substitutions M252Y, S254T, and T256E.

In any of the above embodiments, the hinge-Fc domain of the human immunoglobulin comprises or consists of the amino acid sequence set forth in SEQ ID NO. 3 or 8, or an amino acid sequence having at least 80% sequence identity to the amino acid sequence set forth in SEQ ID NO. 3 or 8, and the hinge-Fc domain of the human immunoglobulin comprises, according to EU numbering, substitutions M252Y, S254T, and T256E. Thus, in some embodiments, the hinge-Fc domain of the human immunoglobulin comprises or consists of the amino acid sequence set forth in SEQ ID NO. 3 or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 3, and the hinge-Fc domain of the human immunoglobulin comprises, according to EU numbering, substitutions M252Y, S254T, and T256E. In some embodiments, the hinge-Fc domain of the human immunoglobulin comprises or consists of the amino acid sequence set forth in SEQ ID NO. 8 or an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to the amino acid sequence set forth in SEQ ID NO. 8, and the hinge-Fc domain of the human immunoglobulin comprises, according to EU numbering, substitutions M252Y, S254T, and T256E.

Thus, in a preferred embodiment, the present invention provides a human VEGF receptor fusion protein, comprising, from N-terminus to C-terminus, an immunoglobulin-like domain 2 of human VEGFR1 whose amino acid sequence is set forth in SEQ ID NO. 1 or 6 or has at least 80% sequence identity thereto, an immunoglobulin-like domain 3 of human VEGFR2 whose amino acid sequence is set forth in SEQ ID NO. 2 or has at least 80% sequence identity thereto, and a hinge-Fc domain of a human immunoglobulin whose amino acid sequence is set forth in SEQ ID NO. 3 or 8 or has at least 80% sequence identity thereto in sequence, wherein the hinge-Fc domain of the human immunoglobulin comprises, according to EU numbering, substitutions M252Y, S254T, and T256E.

In a preferred embodiment, the present invention provides a human VEGF receptor fusion protein, comprising, from N-terminus to C-terminus, an immunoglobulin-like domain 2 of human VEGFR1 whose amino acid sequence is set forth in SEQ ID NO. 1 or 6 or has at least 80% sequence identity thereto, an immunoglobulin-like domain 3 of human VEGFR2 whose amino acid sequence is set forth in SEQ ID NO. 2 or has at least 80% sequence identity thereto, an immunoglobulin-like domain 4 of human VEGFR2 whose amino acid sequence is set forth in SEQ ID NO. 7 or has at least 80% sequence identity thereto, and a hinge-Fc domain of a human immunoglobulin whose amino acid sequence is set forth in SEQ ID NO. 3 or 8 or has at least 80% sequence identity thereto in sequence, wherein the hinge-Fc domain of the human immunoglobulin comprises, according to EU numbering, substitutions M252Y, S254T, and T256E.

In a preferred embodiment, the present invention provides a human VEGF receptor fusion protein, comprising, from N-terminus to C-terminus, an immunoglobulin-like domain 2 of human VEGFR1 whose amino acid sequence is set forth in SEQ ID NO. 1 or has at least 80% sequence identity thereto, an immunoglobulin-like domain 3 of human VEGFR2 whose amino acid sequence is set forth in SEQ ID NO. 2 or has at least 80% sequence identity thereto, and a hinge-Fc domain of a human immunoglobulin whose amino acid sequence is set forth in SEQ ID NO. 3 or has at least 80% sequence identity thereto in sequence, wherein the hinge-Fc domain of the human immunoglobulin comprises, according to EU numbering, substitutions M252Y, S254T, and T256E.

In a preferred embodiment, the present invention provides a human VEGF receptor fusion protein, comprising, from N-terminus to C-terminus, an immunoglobulin-like domain 2 of human VEGFR1 whose amino acid sequence is set forth in SEQ ID NO. 6 or has at least 80% sequence identity thereto, an immunoglobulin-like domain 3 of human VEGFR2 whose amino acid sequence is set forth in SEQ ID NO. 2 or has at least 80% sequence identity thereto, an immunoglobulin-like domain 4 of human VEGFR2 whose amino acid sequence is set forth in SEQ ID NO. 7 or has at least 80% sequence identity thereto, and a hinge-Fc domain of a human immunoglobulin whose amino acid sequence is set forth in SEQ ID NO. 8 or has at least 80% sequence identity thereto in sequence, wherein the hinge-Fc domain of the human immunoglobulin comprises, according to EU numbering, substitutions M252Y, S254T, and T256E.

In a preferred embodiment, the human VEGF receptor fusion protein further comprises a signal peptide at the most N-terminus. One example of signal peptides comprises or consists of the amino acid sequence set forth in SEQ ID NO. 4.

In a preferred embodiment, the present invention provides a human VEGF receptor fusion protein, comprising a hinge-Fc domain of a human immunoglobulin, wherein the human VEGF receptor fusion protein comprises or consists of the amino acid sequence set forth in SEQ ID NO. 5, or an amino acid sequence having at least 80% sequence identity to the amino acid sequence set forth in SEQ ID NO. 5, wherein the hinge-Fc domain of the human immunoglobulin comprises, according to EU numbering, substitutions M252Y, S254T, and T256E.

In a preferred embodiment, the present invention provides a human VEGF receptor fusion protein, comprising a hinge-Fc domain of a human immunoglobulin, wherein the human VEGF receptor fusion protein comprises or consists of the amino acid sequence set forth in SEQ ID NO. 9, or an amino acid sequence having at least 80% sequence identity to the amino acid sequence set forth in SEQ ID NO. 9, wherein the hinge-Fc domain of the human immunoglobulin comprises, according to EU numbering, substitutions M252Y, S254T, and T256E.

In a preferred embodiment, the present invention provides a human VEGF receptor fusion protein, comprising a hinge-Fc domain of a human immunoglobulin, wherein the human VEGF receptor fusion protein is aflibercept, and the hinge-Fc domain of the human immunoglobulin comprises, according to EU numbering, substitutions M252Y, S254T, and T256E.

In a preferred embodiment, the present invention provides a human VEGF receptor fusion protein, comprising a hinge-Fc domain of a human immunoglobulin, wherein the human VEGF receptor fusion protein is conbercept, and the hinge-Fc domain of the human immunoglobulin comprises, according to EU numbering, substitutions M252Y, S254T, and T256E.

In some embodiments, the present invention provides a polynucleotide, such as a DNA or RNA, encoding the human VEGF receptor fusion protein according to any of the above embodiments. In a preferred embodiment, the polynucleotide comprises or consists of the nucleotide sequence set forth in SEQ ID NO. 10 or 11, or a degenerate sequence of any of SEQ ID NOs. 10 and 11. In a preferred embodiment, the polynucleotide is the nucleotide sequence set forth in SEQ ID NO. 10 or 11.

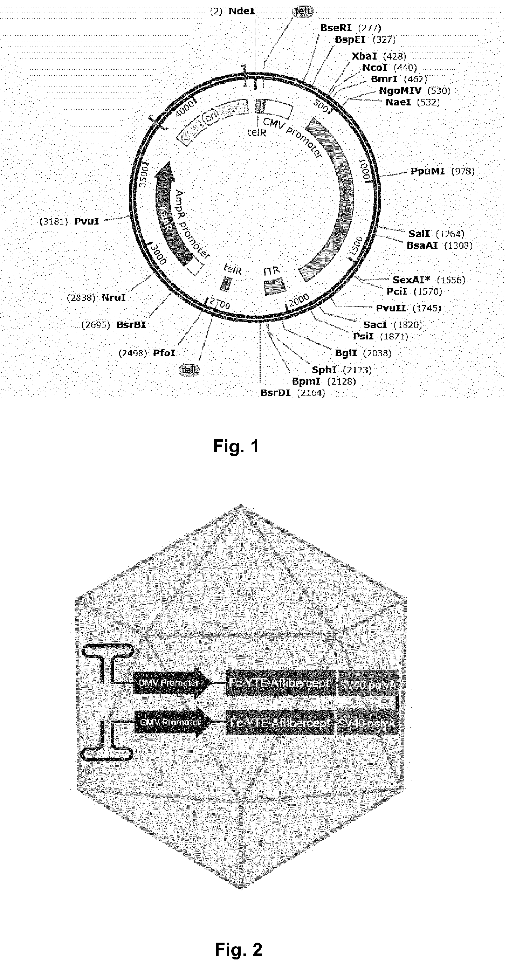

In some embodiments, the present invention provides a vector, such as a plasmid, comprising the polynucleotide. In some embodiments, the plasmid comprises the expression cassette according to any of the above embodiments of expression cassettes. In some aspects, an antibiotic resistance gene is introduced into the plasmid. An antibiotic resistance marker may be used to identify positive transgenic cells in recombinant virus production. In some aspects, the antibiotic marker comprises a sequence encoding the antibiotic resistance gene. For example, markers conferring resistance may include, but are not limited to, kanamycin, gentamicin, ampicillin, chloramphenicol, tetracycline, doxycycline, or hygromycin. In some aspects, the antibiotic resistance gene is a non-beta-lactam antibiotic resistance gene, such as kanamycin. In some embodiments, the vector or plasmid is constructed for formation of an ssAAV, scAAV, or cceAAV. In one embodiment, the plasmid is, for example, the plasmid shown in FIG. 1, which is used to form a cceAAV

In some embodiments, the present invention provides a host cell comprising a vector comprising the polynucleotide, wherein the host cell is non-human, e.g., a HEK293 cell. Other cells such as CHO cells are also possible. In some embodiments, the cell is transfected with the vector comprising the polynucleotide. In addition, the cells are also transfected, for example, with the helper plasmid and accessory plasmid described above simultaneously, wherein the helper plasmid provides, for example, the rep and cap genes of the AAV, and the accessory plasmid provides, for example, genes such as E2, E4a, and/or VA for AAV replication.

### Pharmaceutical Composition, Regimen, and Indications

Another aspect of the present invention provides a pharmaceutical composition, comprising any of the gene delivery vectors (e.g., rAAV) according to the present invention and a pharmaceutically acceptable auxiliary material.

Another aspect of the present invention provides a pharmaceutical composition, comprising any of the human VEGF receptor fusion proteins provided by the present invention, and a pharmaceutically acceptable auxiliary material.

In some embodiments, an rAAV composition is formulated to reduce aggregation of AAV particles in the composition, particularly in the presence of a high rAAV concentration (e.g., ~1013 GC/mL or higher). Methods of reducing rAAV aggregation are well known in the art and include, for example, adding a surfactant, adjusting the pH, adjusting the salt concentration, and the like. Formulation of pharmaceutically acceptable excipient and auxiliary material solutions, as well as the development of suitable administration and treatment regimens for using the particular compositions described herein in a variety of treatment regimens, is well-known to those skilled in the art.

In certain instances, it will be desirable to deliver the rAAV-based therapeutic constructs in suitably formulated pharmaceutical compositions disclosed herein either intravitreally, intraocularly, subretinally, subcutaneously, intrapancreatically, intranasally, parenterally, intravenously, intramuscularly, intrathecally, orally, intraperitoneally, or by inhalation. In some embodiments, the preferred route of administration is by intravenous injection.

Pharmaceutical forms suitable for injection use include sterile aqueous solutions or dispersions and sterile powders for extemporaneous preparation of sterile injectable solutions or dispersions. Dispersions may also be prepared in glycerol, liquid polyethylene glycols, and mixtures thereof and in oils. Under ordinary conditions of storage and use, these formulations contain a preservative to prevent the growth of microorganisms. In many instances, the form is sterile and fluid to the extent that it is easily injected. It must be stable under the conditions of manufacture and storage and must be preserved against the contaminating action of microorganisms, such as bacteria and fungi. The auxiliary material can be a solvent or dispersion medium containing, for example, water, ethanol, polyol (e.g., glycerol, propylene glycol, and liquid polyethylene glycol), suitable mixtures thereof, and/or vegetable oils. Proper fluidity may be maintained, for example, by the use of a coating, such as lecithin, by the maintenance of the required particle size in the case of dispersion, and by the use of surfactants. The action of microorganisms can be prevented by various antibacterial and antifungal agents, for example, parabens, chlorobutanol, phenol, sorbic acid, and thimerosal. In many instances, it will be preferable to include isotonic agents, for example, sugars or sodium chloride. Prolonged absorption of the injectable compositions can be brought about by the use of absorption-delaying agents, such as aluminum monostearate and gelatin, in the compositions.

For administration of an injectable aqueous solution, for example, the solution may be suitably buffered, if necessary, and the liquid diluent is first rendered isotonic with sufficient saline or glucose. These particular aqueous solutions are especially suitable for intravenous, intramuscular, subcutaneous, and intraperitoneal administration. In this regard, a sterile aqueous medium that can be used will be known to those skilled in the art.

Sterile injectable solutions are prepared by incorporating a required amount of the active rAAV and various other ingredients enumerated herein, as needed, into an appropriate solvent, followed by filtration sterilization. Generally, dispersions are prepared by incorporating various sterilized active ingredients into a sterile vehicle that contains the basic dispersion medium and the required other ingredients from those enumerated above. In the case of sterile powders for the preparation of sterile injectable solutions, the preferred preparation methods are vacuum-drying and freeze-drying techniques, which yield a powder of the active ingredient plus any additional required ingredient from a previously sterile-filtered solution thereof.

The rAAV compositions disclosed herein may also be formulated in a neutral or salt form. Pharmaceutically acceptable salts include acid addition salts (formed with the free amino groups of the protein), which are formed with inorganic acids such as hydrochloric acid or phosphoric acid, or organic acids such as acetic acid, oxalic acid, tartaric acid, and mandelic acid. Salts formed with the free carboxyl groups can also be derived from inorganic bases such as sodium hydroxide, potassium hydroxide, ammonium hydroxide, calcium hydroxide, or ferric hydroxide, and organic bases such as isopropylamine, trimethylamine, histidine, and procaine. Upon formulation, solutions will be administered in a manner compatible with the dosage formulation and in a therapeutically effective amount. The formulations are easily administered in a variety of dosage forms such as injectable solutions and drug-release capsules.

As used herein, "auxiliary material" includes any and all solvents, dispersion media, vehicles, coatings, diluents, antibacterial and antifungal agents, isotonic and absorption-delaying agents, buffers, carrier solutions, suspensions, colloids, and the like. The use of such media and agents for pharmaceutical active substances is well known in the art. Supplementary active ingredients may also be incorporated into the compositions. The phrase "pharmaceutically acceptable" refers to molecular entities and compositions that do not produce an allergic or similar untoward reaction when administered to a host.

Delivery vehicles such as liposomes, nanocapsules, microparticles, microspheres, lipid particles, and vesicles may be used for introducing the compositions of the present disclosure into suitable host cells. In particular, the transgene delivered by the rAAV vector may be formulated for delivery, encapsulated in a lipid particle, a liposome, a vesicle, a nanosphere, a nanoparticle, or the like. Such formulations may be preferred for the introduction of pharmaceutically acceptable formulations of the nucleic acids or the rAAV constructs disclosed herein. The formation and use of liposomes are generally known to those skilled in the art. Currently, liposomes with improved serum stability and circulation half-lives have been developed.

In some instances, targeting ocular (e.g., corneal) tissue by intrastromal administration or subcutaneous injection may require different (e.g., higher or lower) doses than by another method (e.g., systemic administration or topical administration). Thus, in some embodiments, the injection is intrastromal injection (IS). In some embodiments, the injection is topical administration (e.g., topical administration to an eye). In some instances, multiple doses of an rAAV are administered.

In some embodiments, administration of an rAAV as described herein results in delivery of a transgene (e.g., KH902) to ocular tissue. The rAAV may be delivered to the ocular tissue of a mammalian subject by, for example, intraocular injection, subretinal injection, or topical administration (e.g., eye drops), or by injection into the eye of the mammalian subject (e.g., intravitreal injection). As used herein, "ocular tissue" refers to any tissue derived from or contained in the eye. Non-limiting examples of ocular tissue include neurons, retina (e.g., photoreceptor cells), sclera, choroid, retina, vitreous body, macula, fovea centralis, optic disc, lens, pupil, iris, aqueous humor, cornea (e.g., keratinocytes, corneal endothelial cells, corneal basal cells, corneal wing cells, and corneal squamous cells), conjunctiva ciliary body, and optic nerve. The retina is located in the posterior of the eye and comprises photoreceptor cells. These photoreceptor cells (e.g., rods and cones) confer visual acuity by discerning color, as well as contrast in the field of view.

Alternatively, the rAAV may be delivered to a mammalian subject by intramuscular injection or by administration into the bloodstream of the mammalian subject. Administration into the bloodstream may be by injection into a vein, an artery, or any other vascular conduit. Non-limiting exemplary methods of intramuscular administration of the rAAV include intramuscular (IM) injection and intravascular infusion. In some embodiments, an rAAV or composition as described in the present disclosure is administered by intravitreal injection. In some embodiments, an rAAV or composition as described in the present disclosure is administered by intraocular injection. In some embodiments, an rAAV or composition as described in the present disclosure is administered by subretinal injection. In some embodiments, an rAAV or composition as described in the present disclosure is administered by intravenous injection. In some embodiments, an rAAV or composition as described in the present disclosure is administered by intramuscular injection.

In some embodiments, administration of an rAAV as described herein results in inhibition of VEGF (e.g., VEGF activity). In some embodiments, administration of an rAAV as described herein results in inhibition of VEGF (e.g., VEGF activity) in ocular tissue. The extent of VEGF inhibition can be measured by any suitable known method (e.g., HUVEC angiogenesis assay, retinal vascular development assay, retinal edema assay, or laser damage-induced choroidal neovascularization (CNV)). In some embodiments, VEGF (e.g., VEGF activity) activity in a subject who has received an anti-VEGF agent (e.g., has been injected with the rAAV described herein) is inhibited by at least 2%, at least 5%, at least 10%, at least 15%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 98%, or at least 100%, compared to an uninjected subject, or the same subject before receiving the anti-VEGF agent. In some embodiments, the VEGF (e.g., VEGF activity) in an uninjected subject or a subject before receiving an anti-VEGF agent is at least 2%, at least 5%, at least 10%, at least 15%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 98%, at least 100%, at least 1-fold, at least 2-fold, at least 3-fold, at least 4-fold, at least 5-fold, at least 6-fold, at least 7-fold, at least 8-fold, at least 9-fold, at least 10-fold, at least 10- to 50-fold (e.g., 10-fold, 20-fold, 30-fold, 40-fold, or 50-fold), or at least 50-to 100-fold (e.g., 50-fold, 60-fold, 70-fold, 80-fold, 90-fold, or 100-fold) higher than that in a subject who has received anti-VEGF agent administration (e.g., has been injected with the rAAV described herein).

In some embodiments, administration of an anti-VEGF agent (e.g., the rAAV described herein) results in inhibition of VEGF (e.g., VEGF activity) for longer than 1 day, longer than 2 days, longer than 3 days, longer than 4 days, longer than 5 days, longer than 6 days, longer than 7 days, longer than 1 week (e.g., 8 days, 9 days, 10 days, 11 days, 12 days, 13 days, or 14 days), longer than 2 weeks (e.g., 15 days, 16 days, 17 days, 18 days, 19 days, 20 days, or 21 days), longer than 3 weeks (e.g., 22 days, 23 days, 24 days, 25 days, 26 days, 27 days, or 28 days), longer than 4 weeks (e.g., 29 days, 30 days, 40 days, 50 days, 60 days, 100 days, or more), longer than 1 month (e.g., 5 weeks, 6 weeks, 7 weeks, 8 weeks, 9 weeks, 10 weeks, or more weeks), longer than 2 months (e.g., between 2 months to 2.5 months, between 2 months to 3 months, between 2 months to 4 months, between 2 months to 5 months, between 2 months to 6 months, between 2 months to 7 months, between 2 months to 8 months, between 2 months to 9 months, between 2 months to 10 months, between 2 months to 11 months, or between 2 months to 12 months), longer than 3 months (e.g., between 3 months to 4 months, between 3 months to 5 months, between 3 months to 6 months, between 3 months to 7 months, between 3 months to 8 months, between 3 months to 9 months, between 3 months to 10 months, between 3 months to 11 months, or between 3 months to 12 months), longer than 4 months (e.g., between 4 months to 5 months, between 4 months to 6 months, between 4 months to 7 months, between 4 months to 8 months, between 4 months to 9 months, between 4 months to 10 months, between 4 months to 11 months, or between 4 months to 12 months), longer than 5 months (e.g., between 5 months to 6 months, between 5 months to 7 months, between 5 months to 8 months, between 5 months to 8 months, between 5 months to 9 months, between 5 months to 10 months, between 5 months to 11 months, or between 5 months to 12 months), longer than 6 months (e.g., between 6 months to 7 months, between 6 months to 8 months, between 6 months to 9 months, between 6 months to 10 months, between 6 months to 11 months, or between 6 months to 12 months), longer than 7 months (e.g., between 7 months to 8 months, between 7 months to 9 months, between 7 months to 10 months, between 7 months to 11 months, or between 7 months to 12 months), longer than 8 months (e.g., between 8 months to 9 months, between 8 months to 10 months, between 8 months to 11 months, or between 8 months to 12 months), longer than 9 months (e.g., between 9 months to 10 months, between 9 months to 11 months, or between 9 months to 12 months), longer than 10 months (e.g., between 10 months to 11 months or between 11 months to 12 months), longer than 11 months (e.g., between 11 months to 12 months), longer than 12 months (e.g., between 12 to 15 months, between 12 to 18 months, between 12 to 21 months, or between 12-2 months), longer than 1 year (e.g., between 1 to 1.5 years), longer than 2 years, longer than 3 years, longer than 4 years, longer than 5 years, longer than 10 years, longer than 15 years, longer than 20 years, or more.

The composition of the present disclosure may comprise an rAAV alone or in combination with one or more other viruses (e.g., a second rAAV having one or more different transgenes). In some embodiments, a composition comprises 1, 2, 3, 4, 5, 6, 7, 8, 9, 10, or more different rAAVs, each of which has one or more different transgenes.

An effective amount of an rAAV or composition is an amount sufficient to infect an animal in a targeting manner or to target a tissue of interest (e.g., muscle tissue or ocular tissue). In some embodiments, the effective amount will depend primarily on factors such as the species, age, weight, health of the subject, and the tissue to be targeted, and may thus vary among animals and tissues. For example, an effective amount of an rAAV is generally in the range from about 1 mL to about 100 mL of solution containing about 10⁶ to 10¹⁶ genome copies (e.g., 1 × 10⁶ to 1 × 10¹⁶, inclusive). In some embodiments, an effective amount of an rAAV ranges between 1 × 10⁹ and 1 × 10¹⁴ rAAV genome copies. In some instances, a dosage of between about 10¹¹ to 10¹² rAAV genome copies is appropriate. In some embodiments, a dosage of between about 10¹¹ to 10¹³ rAAV genome copies is appropriate. In some embodiments, a dosage of between about 10¹¹ to 10¹⁴ rAAV genome copies is appropriate. In some embodiments, a dosage of between about 10¹¹ to 10¹⁵ rAAV genome copies is appropriate. In some embodiments, a dosage of about 10¹² to 10¹⁴ rAAV genome copies is appropriate. In some embodiments, a dosage of about 10¹³ to 10¹⁴ rAAV genome copies is appropriate. In some embodiments, about 1 × 10¹², about 1.1 × 10¹², about 1.2 × 10¹², about 1.3 × 10¹², about 1.4 × 10¹², about 1.5 × 10¹², about 1.6 × 10¹², about 1.7 × 10¹², about 1.8 × 10¹², about 1.9 × 10¹², about 1 × 10¹³, about 1.1 × 10¹³, about 1.2 × 10¹³, about 1.3 × 10¹³, about 1.4 × 10¹³, about 1.5 × 10¹³, about 1.6 × 10¹³, about 1.7 × 10¹³, about 1.8 × 10¹³, about 1.9 × 10¹³, or about 2.0 × 10¹⁴ vector genome (vg) copies per kilogram (kg) of body weight is appropriate. In some embodiments, a dosage of between about 4 × 10¹² to 2 × 10¹³ rAAV genome copies is appropriate. In some embodiments, a dosage of about 1.5 × 10¹³ vg/kg by intravenous administration is appropriate. In certain embodiments, 10¹²-10¹³ rAAV genome copies are effective in the target tissue (e.g., the eye). In certain embodiments, 10¹³-10¹⁴ rAAV genome copies are effective in the target tissue (e.g., the eye).

In some embodiments, the rAAV is injected into the subject. In other embodiments, the rAAV is administrated to the subject by topical administration (e.g., eye drops). In some embodiments, the effective amount of an rAAV is an amount sufficient to express an effective amount of a human VEGF receptor fusion protein in the target tissue (e.g., the eyes) of a subject.

In some embodiments, the effective amount of an rAAV delivered by injection (e.g., delivering an rAAV encoding a human VEGF receptor fusion protein) is an amount sufficient to express an effective amount of a human VEGF receptor fusion protein in the target tissue. In some embodiments, delivery of an effective amount of an rAAV encoding a human VEGF receptor fusion protein is sufficient to deliver 10 µg to 10 mg, or any intermediate value in between, of the human VEGF receptor fusion protein to the subject per eye by suitable routes of administration (e.g., intraocular injection, i.v. injection, intraperitoneal injection, and intramuscular injection). In some embodiments, an rAAV encoding a human VEGF receptor fusion protein is sufficient to deliver 20 µg to 5 mg, or any intermediate value in between, of the human VEGF receptor fusion protein to the subject per eye. In some embodiments, an rAAV encoding a human VEGF receptor fusion protein is sufficient to deliver 10 µg, 20 µg, 30 µg, 40 µg, 50 µg, 60 µg, 70 µg, 80 µg, 90 µg, 100 µg, 200 µg, 300 µg, 400 µg, 500 µg, 600 µg, 700 µg, 800 µg, 900 µg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, or more of the human VEGF receptor fusion protein to the subject per eye.

In some embodiments, an rAAV encoding a human VEGF receptor fusion protein is administered to the subject once a day, once a week, once every two weeks, once a month, once every 2 months, once every 3 months, once every 6 months, once a year, or once in the subject's lifetime.

In some embodiments, the effective amount of an rAAV delivered by topical administration such as eye drops is an amount sufficient to express an effective amount of a human VEGF receptor fusion protein in the target tissue. In some embodiments, eye drops containing an rAAV encoding a human VEGF receptor fusion protein are administered to the subject once a week, once a month, once every 3 months, once every 6 months, or once a year.

In some embodiments, the rAAV encoding a human VEGF receptor fusion protein contained in the eye drops is sufficient to deliver the human VEGF receptor fusion protein at a concentration of 1 mg/mL to 20 mg/mL. In some embodiments, the rAAV encoding a human VEGF receptor fusion protein contained in the eye drops is sufficient to deliver the human VEGF receptor fusion protein at a concentration of 2.5 mg/mL to 10 mg/mL. In some embodiments, the rAAV encoding a human VEGF receptor fusion protein contained in the eye drops is sufficient to deliver the human VEGF receptor fusion protein at a concentration of 1 mg/mL, 2 mg/mL, 2.5 mg/mL, 3 mg/mL, 4 mg/mL, 5 mg/mL, 6 mg/mL, 7 mg/mL, 8 mg/mL, 9 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, or 20 mg/mL. In some embodiments, the eye drops are administered at 0.01 mL, 0.02 mL, 0.03 mL, 0.04 mL, 0.05 mL, 0.06 mL, 0.07 mL, 0.08 mL, 0.09 mL, 0.1 mL, 0.2 mL, 0.3 mL, 0.4 mL, or 0.5 mL.

In some embodiments, the methods described in the present disclosure further comprise the step of inducing immunosuppression (e.g., administering one or more immunosuppressive agents) in a subject before the subject is administered an rAAV (e.g., the rAAV or pharmaceutical composition according to the present disclosure). In some embodiments, a subject is immunosuppressed (e.g., immunosuppression is induced in the subject) between about 30 days to about 0 days (e.g., any time within 30 days before administration of the rAAV, inclusive) before administration of the rAAV to the subject. In some embodiments, the subject is pre-treated with immunosuppressive agents (e.g., rituximab, sirolimus, and/or prednisone) for at least 7 days.

In some embodiments, the methods described in the present disclosure further comprise co-administration or prior administration of an agent to a subject administered the rAAV or pharmaceutical composition comprising the rAAV of the present disclosure. In some embodiments, the agent is selected from Miglustat, Keppra, Prevacid, Clonazepam, and any combination thereof. In some embodiments, the rAAV and the additional agent may be delivered to the subject in any order. In some embodiments, the rAAV and the additional agent (e.g., Miglustat, Keppra, Prevacid, or Clonazepam) are delivered to the subject simultaneously. In some embodiments, the rAAV and the additional agent (e.g., Miglustat, Keppra, Prevacid, or Clonazepam) are co-administered to the subject (e.g., in one composition or in different compositions). In some embodiments, the rAAV is delivered before the additional agent (e.g., Miglustat, Keppra, Prevacid, or Clonazepam). In some embodiments, the rAAV is delivered after the additional agent (e.g., Miglustat, Keppra, Prevacid, or Clonazepam). In some embodiments, the rAAV and the additional agent (e.g., Miglustat, Keppra, Prevacid, or Clonazepam) are delivered to the subject at different frequencies; for example, the subject receives the rAAV every month, every two months, every six months, every year, every two years, every three years, every 5 years, or longer, but receives the additional agent (e.g., Miglustat, Keppra, Prevacid, or Clonazepam) daily, weekly, biweekly, monthly, twice a day, three times a day, or twice a week.

In some embodiments, immunosuppression of the subject is maintained during and/or after administration of the rAAV or pharmaceutical composition. In some embodiments, the subject is immunosuppressed (e.g., administered one or more immunosuppressive agents) for between 1 day to 1 year after administration of the rAAV or pharmaceutical composition.

Another aspect of the present invention provides a method for treating a VEGF-induced disease in a subject, the method comprising administering to the subject a therapeutically effective amount of the rAAV or pharmaceutical composition of the present invention. In a preferred embodiment, the VEGF-induced disease is a disease caused by VEGF overexpression.

In a preferred embodiment, the VEGF-induced disease is selected from exudative (wet) age-related macular degeneration (AMD), diabetic retinopathy (DR) combined with macular edema (DME), retinal vein occlusion (RVO) combined with macular edema (ME), central exudative chorioretinopathy, polypoidal choroidal vasculopathy (PCV), choroidal neovascularization secondary to high myopia, and secondary macular choroidal neovascularization (CNV). In a more preferred embodiment, the VEGF-induced disease is selected from exudative (wet) age-related macular degeneration (AMD), diabetic retinopathy (DR) combined with macular edema (DME), or retinal vein occlusion (RVO) combined with macular edema (ME).

Accordingly, the present invention provides use of the gene delivery vector or the pharmaceutical composition in the preparation of a medicament for treating a VEGF-induced disease. In a preferred embodiment, the VEGF-induced disease is selected from exudative (wet) age-related macular degeneration (AMD), diabetic retinopathy (DR) combined with macular edema (DME), retinal vein occlusion (RVO) combined with macular edema (ME), central exudative chorioretinopathy, polypoidal choroidal vasculopathy (PCV), choroidal neovascularization secondary to high myopia, and secondary macular choroidal neovascularization (CNV). In a more preferred embodiment, the VEGF-induced disease is selected from exudative (wet) age-related macular degeneration (AMD), diabetic retinopathy (DR) combined with macular edema (DME), or retinal vein occlusion (RVO) combined with macular edema (ME).

### Sequence listing

| SEQ ID NO. | Description | Sequence |
|---|---|---|
| 1 | Aflibercept VEGFR1/D2 Aflibercept VEGFR1 immunoglobulin-like domain 2 | SDTGRPFVEMYSEIPEIIHMTEGRELVIPCRVTSPNITVTLKK FPLDTLIPDGKRIIWDSRKGFIISNATYKEIGLLTCEATVNGH LYKTNYLTHRQTNTIID |
| 2 | Aflibercept VEGFR2/D3 Aflibercept VEGFR2 immunoglobulin-like domain 3 | VVLSPSHGIELSVGEKLVLNCTARTELNVGIDFNWEYPSSK HQHKKLVNRDLKTQSGSEMKKFLSTLTIDGVTRSDQGLYT CAASSGLMTKKNSTFVRVHEK |
| 3 | Aflibercept-Fc Aflibercept hinge-Fc | DKTHTCPPCPAPELLGGPSVFLFPPKPKDTLYITREPEVTCV VVDVSHEDPEVKFNWYVDGVEVHNAKTKPREEQYNSTY RVVSVLTVLHQDWLNGKEYKCKVSNKALPAPIEKTISKAK GQPREPQVYTLPPSRDELTKNQVSLTCLVKGFYPSDIAVEW ESNGQPENNYKTTPPVLDSDGSFFLYSKLTVDKSRWQQGN VFSCSVMHEALHNHYTQKSLSLSPG |
| 4 | Signal peptide | MVSYWDTGVLLCALLSCLLLTGSSSG |
| 5 | Fc-YTE-Aflibercept (AA) | MVSYWDTGVLLCALLSCLLLTGSSSGSDTGRPFVEMYSEI PEIIHMTEGRELVIPCRVTSPNITVTLKKFPLDTLIPDGKRII WDSRKGFIISNATYKEIGLLTCEATVNGHLYKTNYLTHRQT NTIIDVVLSPSHGIELSVGEKLVLNCTARTELNVGIDFNWE YPSSKHQHKKLVNRDLKTQSGSEMKKFLSTLTIDGVTRSD QGLYTCAASSGLMTKKNSTFVRVHEKDKTHTCPPCPAPEL LGGPSVFLFPPKPKDTLYITREPEVTCVVVDVSHEDPEVKF NWYVDGVEVHNAKTKPREEQYNSTYRVVSVLTVLHQDW LNGKEYKCKVSNKALPAPIEKTISKAKGQPREPQVYTLPPS RDELTKNQVSLTCLVKGFYPSDIAVEWESNGQPENNYKTT PPVLDSDGSFFLYSKLTVDKSRWQQGNVFSCSVMHEALH NHYTQKSLSLSPG |
| 6 | Conbercept R1/D2 Conbercept VEGFR1 immunoglobulin-like domain 2 | GRPFVEMYSEIPEIIHMTEGRELVIPCRVTSPNITVTLKKFPL DTLIPDGKRIIWDSRKGFIISNATYKEIGLLTCEATVNGHLY KTNYLTHRQTNTIID |
| 7 | Conbercept R2/D4 Conbercept VEGFR2 immunoglobulin-like domain 4 | PFVAFGSGMESLVEATVGERVRIPAKYLGYPPPEIKWYKNG IPLESNHTIKAGHVLTIMEVSERDTGNYTVILTNPISKEKQS HVVSLVVYVPPGPG |
| 8 | Conbercept-Fc Conbercept hinge-Fc | DKTHTCPLCPAPELLGGPSVFLFPPKPKDTLYITREPEVTCV VVDVSHEDPEVKFNWYVDGVEVHNAKTKPREEQYNSTY RVVSVLTVLHQDWLNGKEYKCKVSNKALPAPIEKTISKAK GQPREPQVYTLPPSRDELTKNQVSLTCLVKGFYPSDIAVEW |
| | | ESNGQPENNYKATPPVLDSDGSFFLYSKLTVDKSRWQQGN VFSCSVMHEALHNHYTQKSLSLSPGK |
| 9 | Fc-YTE-Conbercept (AA) | MVSYWDTGVLLCALLSCLLLTGSSSGGRPFVEMYSEIPEII HMTEGRELVIPCRVTSPNITVTLKKFPLDTLIPDGKRIIWDS RKGFIISNATYKEIGLLTCEATVNGHLYKTNYLTHRQTNTII DVVLSPSHGIELSVGEKLVLNCTARTELNVGIDFNWEYPSS KHQHKKLVNRDLKTQSGSEMKKFLSTLTIDGVTRSDQGLY TCAASSGLMTKKNSTFVRVHEKPFVAFGSGMESLVEATVG ERVRIPAKYLGYPPPEIKWYKNGIPLESNHTIKAGHVLTIM EVSERDTGNYTVILTNPISKEKQSHVVSLVVYVPPGPGDKT HTCPLCPAPELLGGPSVFLFPPKPKDTLYITREPEVTCVVVD VSHEDPEVKFNWYVDGVEVHNAKTKPREEQYNSTYRVV SVLTVLHQDWLNGKEYKCKVSNKALPAPIEKTISKAKGQP REPQVYTLPPSRDELTKNQVSLTCLVKGFYPSDIAVEWESN GQPENNYKATPPVLDSDGSFFLYSKLTVDKSRWQQGNVFS CSVMHEALHNHYTQKSLSLSPGK |
| 10 | Fc-YTE-Aflibercept(DNA) | Atggtgtcctactgggacacaggcgtgctgttgtgcgccctgctgtcttgtctgctgctgaca ggatctagcagcggcagcgatacaggccggccttttgtggaaatgtactctgaaatccccga gatcatccacatgaccgagggcagagagctggtgatcccctgcagagtcacatcccccaa catcaccgtgaccctgaagaaattccccctggataccctgatccctgacggcaagagaatca tctgggatagcagaaaaggctttatcatcagcaacgccacctacaaggaaatcggcctgctg acctgcgaggccactgtgaacggccacctgtacaagacaaactacctgacacacagacag accaacaccatcattgatgtggtgctctctcctagccacggcatcgagctgtctgtgggcga gaaactggtgctgaactgcaccgctagaaccgagcttaatgtgggaatcgacttcaattggg agtatccatcaagtaagcaccaacacaagaagctggtgaacagggacctgaaaacacaga gtggctctgagatgaaaaagttcctgagcaccctgacaatcgacggagttacaagaagcga ccagggcctgtacacctgtgccgccagcagcggcctgatgaccaagaagaatagcacattt gtgcgggtgcacgagaaggacaagacccacacctgcccaccatgtcctgctcctgagctg ctgggcggccctagcgtgttcctgttccctcctaagcccaaggataccctgtacatcaccag agagcccgaggtgacctgcgtggtggtcgacgtgtctcatgaagatcctgaggtgaagttc aactggtacgtggacggagttgaagtgcacaatgccaagaccaagcctagagaggaacag tacaacagcacctaccgggtggtcagcgtgctgaccgtcctgcaccaggactggctgaac ggcaaggaatacaaatgcaaggtgtccaacaaggccctgcccgcccctatcgagaagacg attagcaaagctaagggacagcctcgggaaccccaggtgtacaccctgcctccaagccgg gacgagctgacaaagaaccaggtgtccctgacatgtctggtcaagggcttctacccctctga tatcgccgtggaatgggaaagcaacggccaacctgaaaacaactacaaaacaacccctcct gtgctggacagcgacggcagcttcttcctgtatagcaagctgaccgtggataagagccgct |
| | | ggcagcagggcaacgtgttcagctgcagcgtgatgcacgaggcccttcataatcactacac ccagaagtccctgagcctgagccctggctaa |
| 11 | Fc-YTE-Conbercept (DNA) | atggtcagctactgggacaccggggtcctgctgtgcgcgctgctcagctgtctgcttctcaca ggatctagttccggaggtagacctttcgtagagatgtacagtgaaatccccgaaattatacac atgactgaaggaagggagctcgtcattccctgccgggttacgtcacctaacatcactgttact ttaaaaaagtttccacttgacactttgatccctgatggaaaacgcataatctgggacagtagaa agggcttcatcatatcaaatgcaacgtacaaagaaatagggcttctgacctgtgaagcaaca gtcaatgggcatttgtataagacaaactatctcacacatcgacaaaccaatacaatcatagat gtggttctgagtccgtctcatggaattgaactatctgttggagaaaagcttgtcttaaattgtaca gcaagaactgaactaaatgtggggattgacttcaactgggaatacccttcttcgaagcatcag cataagaaacttgtaaaccgagacctaaaaacccagtctgggagtgagatgaagaaattttt gagcaccttaactatagatggtgtaacccggagtgaccaaggattgtacacctgtgcagcat ccagtgggctgatgaccaagaagaacagcacatttgtcagggtccatgaaaaaccttttgttg cttttggaagtggcatggaatctctggtggaagccacggtgggggagcgtgtcagaatccct gcgaagtaccttggttacccacccccagaaataaaatggtataaaaatggaataccccttga gtccaatcacacaattaaagcggggcatgtactgacgattatggaagtgagtgaaagagac acaggaaattacactgtcatccttaccaatcccatttcaaaggagaagcagagccatgtggtc tctctggttgtgtatgtcccaccgggcccgggcgacaaaactcacacatgcccactgtgccc agcacctgaactcctggggggaccgtcagtcttcctcttccccccaaaacccaaggacacc ctctacatcacccgggagcctgaggtcacatgcgtggtggtggacgtgagccacgaagac cctgaggtcaagttcaactggtacgtggacggcgtggaggtgcataatgccaagacaaagc cgcgggaggagcagtacaacagcacgtaccgtgtggtcagcgtcctcaccgtcctgcacc aggactggctgaatggcaaggagtacaagtgcaaggtctccaacaaagccctcccagccc ccatcgagaaaaccatctccaaagccaaagggcagccccgagaaccacaggtgtacacc ctgcccccatcccgggatgagctgaccaagaaccaggtcagcctgacctgcctagtcaaa ggcttctatcccagcgacatcgccgtggagtgggagagcaatgggcagccggagaacaa ctacaaggccacgcctcccgtgctggactccgacggctccttcttcctctacagcaagctca ccgtggacaagagcaggtggcagcaggggaacgtcttctcatgctccgtgatgcatgagg ctctgcacaaccactacacgcagaagagcctctccctgtctccgggtaaatga |

### Examples

### Example 1. Preparation of rAAVFc-YTE-Aflibercept

The obtained Fc-YTE-aflibercept gene sequence (SEQ ID NO. 10) was loaded onto an AAV master plasmid (a GOI plasmid) (the plasmid map is shown in FIG. 1), and with the help of an auxiliary plasmid (such as a helper plasmid or R/C plasmid), a corresponding recombinant adeno-associated virus (rAAV) drug rAAV-Fc-YTE-aflibercept (also referred to as "rAAV-Fc-YTE") was prepared. In this example, the master plasmid backbone used was of a cceAAV genomic configuration; the promoter element used was a CMV promoter sequence; the PolyA element used was an SV40 late PolyA sequence; and the viral capsid used was of an AAV8 serotype.

AAV virus packaging production was performed using a three-plasmid system, and it was completed by transient transfection of HEK293 cells with the following three-plasmid system. The three plasmids and their functions are described below.

Master plasmid (GOI plasmid): As described above, the vector genomic DNA containing the Fc-YTE-aflibercept gene sequence expression cassette, after being administered, can express the Fc-YTE-aflibercept protein molecule (the amino acid sequence of which is set forth in SEQ ID NO. 5) in target tissues *in vivo.*

R/C plasmid (helper plasmid): for providing in trans Rep and Cap proteins of the rAAV. The R/C plasmid gene and the master plasmid gene were placed in two independent plasmid systems. This arrangement ensures that Rep and Cap structures are expressed exclusively in the rAAV, thereby eliminating the replication function of the rAAV.

Accessory plasmid: loaded with gene segments essential for starting AAV replication in an adenovirus, such as E2, E4a, and VA. The helper plasmid, the GOI master plasmid, and the R/C plasmid were co-transfected into a HEK293 cell. Through the combined action of these plasmids and packaging, an rAAV-Fc-YTE-aflibercept virus was prepared. A schematic diagram of the structure of the virus is shown in FIG. 2. Subsequently, related quality tests were performed, mainly including tests for the viral genome titer (Vg), the capsid protein purity, the number of virus particles (Vp), host cell DNA residue (HCD), host cell protein residue (HCP), bacterial endotoxin, etc. It was confirmed that all the corresponding requirements were met.

### Example 2. rAAV Fc-YTE-Aflibercept Causes Significant Reductions in FFA Spot Score and Area

The rAAV-Fc-YTE-aflibercept provided by Example 1 was subjected to efficacy verification, efficacy evaluation, and comparison using a laser-induced C57BL/6J mouse (male, about 6 weeks) CNV model.
1) Groups and administration regimens
   Negative control: a negative control reagent (the vehicle reagent Buffer)
   Positive control 1: a positive drug molecule (a commercially available clinical aflibercept protein injection)
   Positive control 2: rAAV-Eylea
   Experimental group: rAAV-Fc-YTE
   Compared to the rAAV-Fc-YTE group, the Fc fragment of IgG1 of Eylea (the trade name of aflibercept) of the rAAV-Eylea group was a wild-type Fc without the YTE site-directed mutagenesis optimization. Both used the same master plasmid expression framework and regulatory elements and were prepared using the method described in Example 1.

### Groups and administration regimens

| | Test substance group | Number of animals | Dose | Injection site |
|---|---|---|---|---|
| G1 | rAAV-Eylea | 6 | 1×10⁹ vg/µL/eye | SRI, bilateral |
| G2 | rAAV-Fc-YTE | 6 | 1×10⁹ vg/µL/eye | SRI, bilateral |
| G3 | Negative control reagent | 6 | 1 µL | SRI, bilateral |
| G4 | Positive drug molecule | 6 | 20 µg/eye | IVT, bilateral |

| | | | | |
|---|---|---|---|---|
| Note: Modeling was performed 4 weeks after administration, and the day of modeling was denoted by day 0. | | | | |

At day -28, the animals were anesthetized with Zoletil (25-50 mg/kg, i.p.) + a xylazine hydrochloride injection (5 mg/kg, i.p.) before administration. The animals in the groups G1-G3 were bilaterally and subretinally injected once with different doses of test substances and Buffer (1 µL/eye). At day 3 after modeling, the animals in the group G4 were intravitreally injected with the positive drug (0.5 µL/eye).

Subretinal injection: The ocular surface was disinfected with iodophor, and under an ophthalmic surgical microscope, a 30G disposable syringe needle was used to puncture the sclera on the inner side of the corneoscleral limbus in the mice. A microliter syringe with a 35G blunt needle was inserted through the puncture, bypassing the lens to reach the vitreous body while avoiding the main blood vessels. The needle was then gradually advanced into the subretinal space, and the test sample was slowly injected. Immediately after the syringe needle was pulled out, the puncture was pressed with a cotton swab for 5 s. Immediately after administration, an OCT examination was performed to check if the injection was successful. If a noticeable elevation of the retina is observed under OCT, it indicates that the administration has been successful.

Care after administration: After administration, all the animals enrolled were treated with levofloxacin eye drops and ofloxacin eye ointment in both eyes, once in the morning and once in the afternoon, for three consecutive days.

### 2) Modeling:

At day 0 (4 weeks after administration), the animals were anesthetized with Zoletil (25-50 mg/kg, i.p.) and a xylazine hydrochloride injection (5 mg/kg, i.p.). Using a YAG laser photocoagulator (VITRA, Quantel Medical), 3 laser burns were made on the RPE/Bruch's membrane in both eyes with a 532 nm laser at the same energy setting (3 photocoagulation spots were evenly distributed around the optic nerve head 1-1.5 PD away from the optic disc). The laser burns should be made while avoiding major retinal vessels and the injection site to prevent intraocular bleeding.

Mark of success in making laser burns: Immediately after the laser treatment, an OCT examination was performed to locate the laser burns and check if the laser burns and visible ruptures in the Bruch's membrane were successfully made.

### 3) Main indicators and results:

### 3.1) Clinical observation:

Cage-side observation was performed once daily to see if there were dead animals and to observe the animals' mental states, behavior, activity, and the like.

Results: According to the once-daily cage-side observations of the animals enrolled, no significantly abnormal clinical symptoms were observed.

### 3.2) SD-OCT:

OCT examinations were performed using ultra-high resolution spectral-domain optical coherence tomography (SD-OCT). The mice were anesthetized, their pupils were dilated, and the mice were positioned in such a way as to allow the optic nerve head (ONH) to appear at the center of the image. B-scans (5 frames on average) and full-field volumetric scans (300 frames) were captured.

SD-OCT scanning was performed on both eyes of each of the animals immediately after day -28 administration (the rAAV-Eylea group, the rAAV-Fc-YTE group, and the negative control reagent group) and immediately after day 0 modeling (the rAAV-Eylea group, the rAAV-Fc-YTE group, the negative control reagent group, and the positive drug molecule group).

Conclusion: No abnormal clinical symptoms were observed in the animals enrolled throughout the experiment. The OCT scanning results show that immediately after day -28 administration (the rAAV-Eylea group, the rAAV-Fc-YTE group, and the negative control reagent group), the retinas of the animals were all visibly elevated, which indicates that the subretinal injections were successfully administered; immediately after day 0 modeling (the rAAV-Eylea group, the rAAV-Fc-YTE group, the negative control reagent group, and the positive drug molecule group), visible laser burns and ruptures in the Bruch's membrane were observed in all the animals, which indicates that the laser modeling was a success.

### 3.3) FFA spot score and spot area:

At day 7 after modeling, all the animals were subjected to FFA testing. Before testing, the animals were anesthetized with Zoletil (25-50 mg/kg, i.p.) + a xylazine hydrochloride injection (5 mg/kg, i.p.). After injection of fluorescein sodium, radiographic images were taken successively. Leakage scores were recorded in the early stage and the late stage after injection of fluorescein sodium. Leakages were graded on a scale of I to IV, as shown in the table below.

### Details of FFA scoring

| | |
|---|---|
| Grade I | No strong fluorescence. |
| Grade II | The foci are highly fluorescent in the early or intermediate stage and do not leak. |
| Grade III | The foci are highly fluorescent in the early stage and leak in the late stage. |
| Grade IV | The foci are brightly and highly fluorescent in the early stage and leak beyond the boundaries of the burns in the late stage. |

### Results:

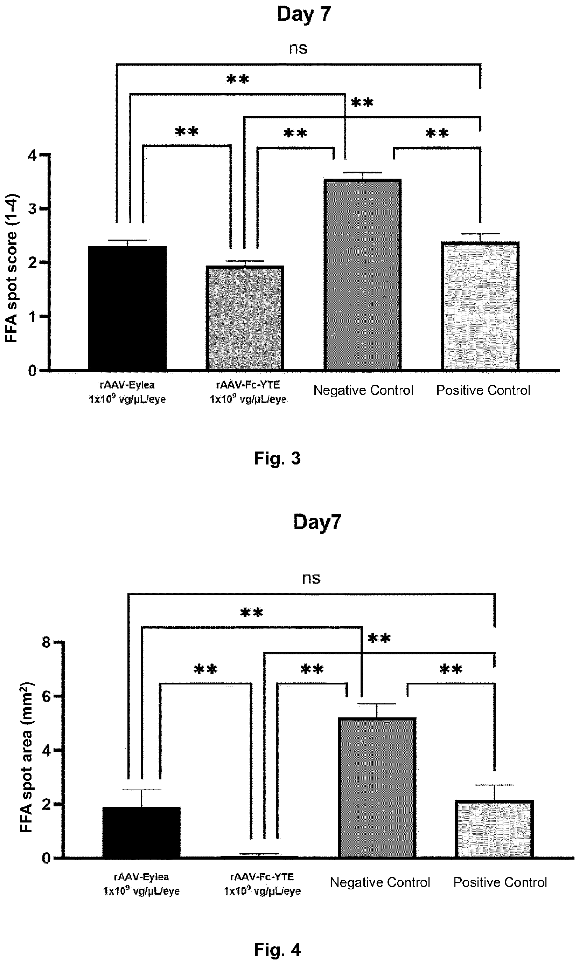

At day 7 after modeling, all the animals were subjected to FFA testing. The FFA scores and area measurements are shown in FIGs. 3 and 4, respectively, and are summarized below. The FFA spot area was used to evaluate efficacy. The smaller the spot area, the better the efficacy.

### FFA spot score (mean ± SEM)

| Group test substance | Number of eyes | Post-modeling day 7 spot score |
|---|---|---|
| rAAV-Eylea | n=12 | 2.31±0.10 |
| rAAV-Fc-YTE | n=12 | 1.94±0.08 |
| Negative control reagent | n=12 | 3.56±0.11 |
| Positive drug molecule | n=12 | 2.39±0.14 |

### FFA spot area (mm², mean ± SEM)

| Test substance group | Number of eyes | Post-modeling day 7 spot area |
|---|---|---|
| rAAV-Eylea | n=12 | 1.90±0.63 |
| rAAV-Fc-YTE | n=12 | 0.08±0.08 |
| Negative control reagent | n=12 | 5.22±0.50 |
| Positive drug molecule | n=12 | 2.15±0.57 |

Brief summary: The post-modeling day 7 fundus fluorescein angiography (FFA) results show that:

a. compared to the negative control reagent group, the positive drug molecule group (positive control 1), the rAAV-Eylea group (positive control 2), and the rAAV-Fc-YTE group (experimental group) all showed significant decreases in spot score and spot area; and

b. compared to the positive drug molecule group (positive control 1) and the rAAV-Eylea group (positive control 2), the rAAV-Fc-YTE group (experimental group) showed a significant decrease in FFA spot area.

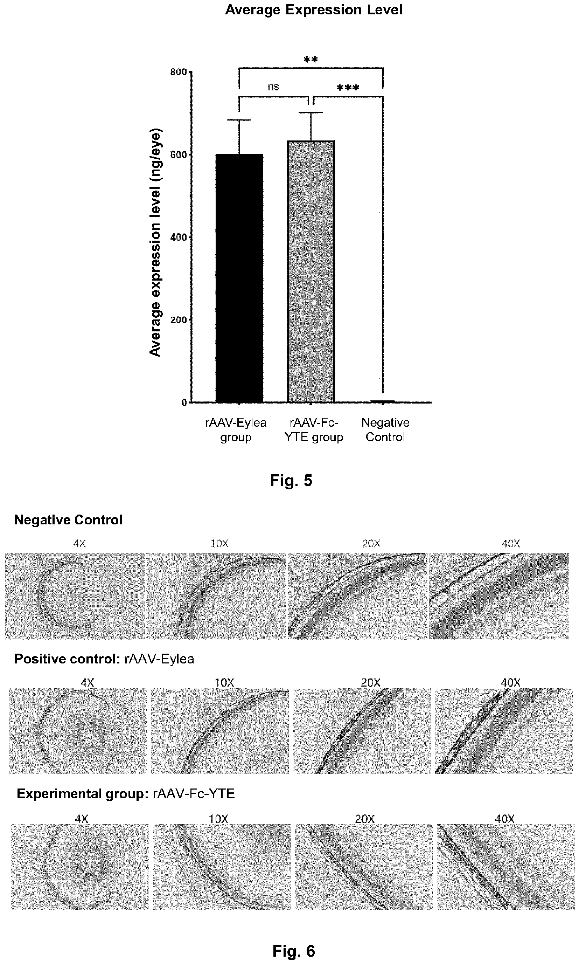

3.4) Elisa assay: At day 14 after modeling (42 days after injection), 3 eyeballs were randomly selected from each of the negative control reagent group, the rAAV-Eylea group (positive control 2), and the rAAV-Fc-YTE group (experimental group) and assayed by Elisa for the expression distribution level of the drug protein molecule of each of the groups. The results are shown in FIG. 5 and summarized below.

| Group No. | Animal No. | Eye | Protein expression level ng/eye | Average expression level ng/eye | SEM |
|---|---|---|---|---|---|
| rAAV-Eylea group | 39542 | OD | 466.77 | 602.25 | 81.63 |
| | 39544 | OD | 591.09 | | |
| | 39545 | OD | 748.89 | | |
| rAAV-Fc-YTE group | 39510 | OD | 667.43 | 634.86 | 66.83 |
| | 39526 | OD | 506.32 | | |
| | 39530 | OD | 730.84 | | |
| Negative control reagent group | 39573 | OD | 4.30 | 1.13 | 2.07 |
| | 39576 | OD | 1.84 | | |
| | 39579 | OD | 2.76 | | |

### Brief summary:

The rAAV-Eylea group (positive control 2) and the rAAV-Fc-YTE group (experimental group) both showed relatively high protein expression levels in target tissue mouse eyeballs, and their protein expression levels were similar.

### 3.5) Statistical analysis:

Experimental data are expressed using mean ± standard error of the mean (mean ± S.E.M.). Data were analyzed by Graphpad Prism or SPSS using the corresponding statistical methods. Kruskal-Wallis test/Dunnett's test (for comparisons between multiple groups) and Mann-Whitney test (for comparisons between two groups) were used to analyze FFA spot score and spot area data, and One-Way ANOVA was used to comparatively analyze protein expression data; P < 0.05 was considered to indicate a significant difference.

### 3.6) Summary of conclusions:

A. In the example, compared to the negative control group, the experimental group and the 2 positive control groups all showed significant differences and efficacy; positive control 1 (intravitreal injection of Eylea) and positive control 2 (subretinal injection of rAAV-Eylea) were similar in efficacy, further indicating that the modeling experimental system was a success.

B. There was no difference in drug protein expression level in target tissue between the rAAV-Fc-YTE group (experimental group) and positive control 2 (subretinal injection of rAAV-Eylea) of the example of the present invention, but the experimental group showed significant and better efficacy than both the positive control groups.

### Example 3. Expression Distributions of Drug Molecules in Target Tissue

1) Experimental design: The same test samples as those used in Example 2 were adopted and injected into C57BL/6J mice using the same subretinal injection mode and dosage (1 × 10⁹ vg/pL/eye, SRI, bilateral) as those used in Example 2. At day 33, eyeball samples were collected from the mice and embedded in paraffin, and the expression distributions of the genes of the drug proteins of interest across various layers of retinal cells were measured using BaseScope technology at the mRNA level (given that the proteins of interest were secreted proteins, BaseScope technology was used to measure transcription levels instead of using IHC technology to measure protein levels).
   Negative control: a negative control reagent (the vehicle reagent Buffer)
   Positive control: rAAV-Eylea
   Experimental group: rAAV-Fc-YTE (CRG-B191)
   Representative BaseScope results are shown in FIG. 6.

Conclusion: The results show that the positive control (rAAV-Eylea) and the experimental group (rAAV-Fc-YTE) both showed efficient expression in the target tissue as compared to the negative control (the negative control reagent). Moreover, the expression distribution of the positive control drug gene across the various layers of the structure of the retinal was the same as that of the experimental group drug gene: they were expressed in cells of the RPE layer, the IS/OS layer, and the outer nuclear layer of the retina, with the expression level in RPE cells being the highest.

The above results show that the engineering of the drug molecule in the present invention can significantly improve the therapeutic effect on diseases of interest without changing the expression level and expression distribution of the drug molecule in the target tissue.

### Example 4. Recombinant Protein Drug CNV Modeling and FFA Testing

Modeling and FFA scoring and testing were performed using the methods described in Example 2. Protein drug groups and administration regimens are shown in the table below. The results show that the efficacy of direct intraocular IVT administration of the Fc-YTE-aflibercept protein was not inferior to that of aflibercept.

### Groups and administration regimens

| Group No. | Test substance | Number of animals | Dose | Mode of injection | Treatment method and time |
|---|---|---|---|---|---|
| 1 | G1: negative control (PBS) | 6 | 2ul/eye | IVT, bilateral | Modeling at day 0, single administration at day 3, FFA testing at day 7 |
| 2 | G2: aflibercept | 6 | 2 µL/eye (diluted with PBS to the same concentration as the test substance) | IVT, bilateral | Modeling at day 0, single administration at day 3, FFA testing at day 7 |
| 3 | G3: test substance (Fc-YTE-aflibercept) | 6 | 2ul/eye (6.34mg/ml) | IVT, bilateral | Modeling at day 0, single administration at day 3, FFA testing at day 7 |

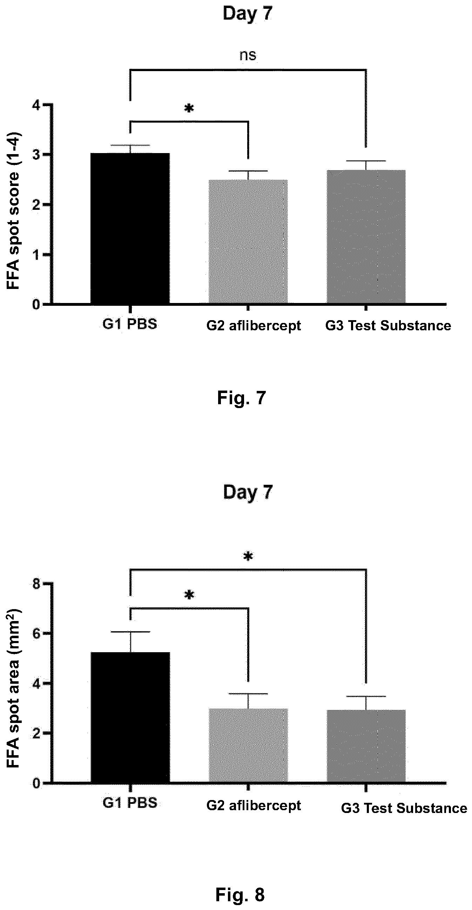

The post-modeling day 7 FFA spot scores (mean ± SEM) are shown in the table below and FIG. 7.

| Group test substance | Number of eyes | Post-modeling day 7 spot score |
|---|---|---|
| G1: negative control (PBS) | n=12 | 3.03±0.16 |
| G2: aflibercept | n=12 | 2.50±0.18 |
| G3: test substance (Fc-YTE-aflibercept) | n=12 | 2.69±0.18 |

The post-modeling day 7 FFA spot areas (mm², mean ± SEM) are shown in the table below and FIG. 8.

| Test substance group | Number of eyes | Post-modeling day 7 spot area |
|---|---|---|
| G1: negative control (PBS) | n=12 | 5.25±0.82 |
| G2: aflibercept | n=12 | 2.99±0.60 |
| G3: test substance (Fc-YTE-aflibercept) | n=12 | 2.94±0.55 |

## Claims

1. A gene delivery vector, comprising a nucleic acid, wherein the nucleic acid comprises an expression cassette that expresses a human VEGF receptor fusion protein; the human VEGF receptor fusion protein comprises an immunoglobulin-like domain 2 of human VEGFR1, an immunoglobulin-like domain 3 of human VEGFR2, and a hinge-Fc domain of a human immunoglobulin; the hinge-Fc domain of the human immunoglobulin comprises, according to EU numbering, substitutions M252Y, S254T, and T256E.

2. The gene delivery vector according to claim 1, wherein:
(a) the human VEGF receptor fusion protein further comprises an immunoglobulin-like domain 4 of human VEGFR2; and/or
(b) the hinge-Fc domain of the human immunoglobulin is a hinge-Fc domain of human IgG1; and/or
(c) the immunoglobulin-like domain 2 of human VEGFR1 comprises or consists of the amino acid sequence set forth in SEQ ID NO. 1 or 6, or an amino acid sequence having at least 80% sequence identity to the amino acid sequence set forth in SEQ ID NO. 1 or 6; and/or
(d) the immunoglobulin-like domain 3 of human VEGFR2 comprises or consists of the amino acid sequence set forth in SEQ ID NO. 2, or an amino acid sequence having at least 80% sequence identity to the amino acid sequence set forth in SEQ ID NO. 2; and/or
(e) the immunoglobulin-like domain 4 of human VEGFR2 comprises or consists of the amino acid sequence set forth in SEQ ID NO. 7, or an amino acid sequence having at least 80% sequence identity to the amino acid sequence set forth in SEQ ID NO. 7; and/or
(f) the hinge-Fc domain of the human immunoglobulin comprises or consists of the amino acid sequence set forth in SEQ ID NO. 3 or 8, or an amino acid sequence having at least 80% sequence identity to the amino acid sequence set forth in SEQ ID NO. 3 or 8.

3. The gene delivery vector according to claim 1, wherein the human VEGF receptor fusion protein comprises or consists of the amino acid sequence set forth in SEQ ID NO. 5 or 9, or an amino acid sequence having at least 80% sequence identity to the amino acid sequence set forth in SEQ ID NO. 5 or 9.

4. The gene delivery vector according to any one of claims 1 to3, wherein:
(a) the expression cassette comprises: the nucleotide sequence set forth in SEQ ID NO. 10 or 11, or a degenerate sequence of any of SEQ ID NOs. 10 and 11; and/or
(b) the expression cassette further comprises a promoter that is upstream of a nucleotide sequence encoding the human VEGF receptor fusion protein and is operably linked thereto; the promoter is preferably selected from a cytomegalovirus (CMV) promoter, a Rous sarcoma virus (RSV) promoter, an MMT promoter, an EF-1α promoter, a U6 promoter, a chicken β-actin promoter, a CAG promoter, a CBA promoter, an RPE65 promoter, a VMD2 promoter, an RPGR promoter, an IRBP promoter, an hGRK1 promoter, a CAR promoter, an RHO promoter, a Grm6 promoter, a GRK1 promoter, and a GFAP promoter; and/or
(c) the expression cassette further comprises a polyadenylation signal that is downstream of the nucleotide sequence encoding the human VEGF receptor fusion protein and is operably linked thereto; the polyadenylation signal is preferably selected from an SV40 early poly A, an SV40 late polyA, a rabbit globin polyA, a bGH polyA, and an HSV TK polyA.

5. The gene delivery vector according to any one of claims 1 to 4, wherein the gene delivery vector is
(a) a viral vector, preferably a lentivirus, retrovirus, adenovirus, adeno-associated virus, herpesvirus, poxvirus, papovavirus, baculovirus, or papillomavirus, and more preferably an adeno-associated virus or lentivirus; or
(b) a non-viral vector, preferably a plasmid, liposome, nanoparticle, polymer, transposon, exosome, or bacterial vector;

6. The gene delivery vector according to any one of claims 1 to 5, wherein the vector is an adeno-associated virus vector, comprising: (a) a recombinant adeno-associated virus capsid, and (b) the nucleic acid packaged within the recombinant adeno-associated virus capsid;
preferably, wherein
(a) the recombinant adeno-associated virus capsid comprises capsid proteins of an adeno-associated virus selected from the following serotypes: AAV1, AAV2, AAV3, AAV4, AAV5, AAV6, AAV7, AAV8, AAV9, AAV10, AAV11, AAV12, and hybrids thereof, and capsid protein mutants obtained by engineering with the above capsid proteins as backbones; preferably, the recombinant adeno-associated virus capsid comprises capsid proteins of an adeno-associated virus selected from AAV1, AAV2, AAV5, AAV7, AAV8, and hybrids thereof; and/or
(b) the genome of the adeno-associated virus vector is single-stranded or doublestranded and is preferably an scAAV, ssAAV, or cceAAV.

7. A pharmaceutical composition, comprising the gene delivery vector according to any one of claims 1 to 6 and a pharmaceutically acceptable excipient; preferably, the pharmaceutical composition is an injectable formulation.

8. The gene delivery vector according to any one of claims 1 to 6 or the pharmaceutical composition according to claim 7 for use in treating a VEGF-induced disease, wherein preferably the VEGF-induced disease is selected from exudative (wet) age-related macular degeneration (AMD), diabetic retinopathy (DR) combined with macular edema (DME), retinal vein occlusion (RVO) combined with macular edema (ME), central exudative chorioretinopathy, polypoidal choroidal vasculopathy (PCV), choroidal neovascularization secondary to high myopia, and secondary macular choroidal neovascularization (CNV).

9. A human VEGF receptor fusion protein, comprising an immunoglobulin-like domain 2 of human VEGFR1, an immunoglobulin-like domain 3 of human VEGFR2, and a hinge-Fc domain of a human immunoglobulin, wherein the hinge-Fc domain of the human immunoglobulin comprises, according to EU numbering, substitutions M252Y, S254T, and T256E;
preferably, wherein
(a) the human VEGF receptor fusion protein further comprises an immunoglobulin-like domain 4 of human VEGFR2; preferably, the immunoglobulin-like domain 4 of human VEGFR2 comprises or consists of the amino acid sequence set forth in SEQ ID NO. 7, or an amino acid sequence having at least 80% sequence identity to the amino acid sequence set forth in SEQ ID NO. 7; and/or
(b) the hinge-Fc domain of the human immunoglobulin is a hinge-Fc domain of human IgG1; preferably, the hinge-Fc domain of human IgG1 comprises or consists of the amino acid sequence set forth in SEQ ID NO. 3 or 8, or an amino acid sequence having at least 80% sequence identity to the amino acid sequence set forth in SEQ ID NO. 3 or 8; and/or
(c) the immunoglobulin-like domain 2 of human VEGFR1 comprises or consists of the amino acid sequence set forth in SEQ ID NO. 1 or 6, or an amino acid sequence having at least 80% sequence identity to the amino acid sequence set forth in SEQ ID NO. 1 or 6; and/or
(d) the immunoglobulin-like domain 3 of human VEGFR2 comprises or consists of the amino acid sequence set forth in SEQ ID NO. 2, or an amino acid sequence having at least 80% sequence identity to the amino acid sequence set forth in SEQ ID NO. 2.

10. The human VEGF receptor fusion protein according to claim 9, wherein the human VEGF receptor fusion protein comprises or consists of the amino acid sequence set forth in SEQ ID NO. 5 or 9, or an amino acid sequence having at least 80% sequence identity to the amino acid sequence set forth in SEQ ID NO. 5 or 9.

11. A polynucleotide encoding the human VEGF receptor fusion protein according to claim 9 or 10, wherein preferably, the polynucleotide comprises or consists of the nucleotide sequence set forth in SEQ ID NO. 10 or 11, or a degenerate sequence of any of SEQ ID NOs. 10 and 11.

12. A vector comprising the polynucleotide according to claim 11, wherein preferably, the vector is a plasmid; preferably, the vector is constructed for formation of an ssAAV, scAAV, or cceAAV.

13. A host cell comprising the vector according to claim 12, wherein preferably, the host cell is a HEK293 cell or sf9 cell.

14. A recombinant adeno-associated virus, comprising:
(a) an rAAV capsid; and
(b) a nucleic acid packaged within the rAAV capsid, the nucleic acid comprising in 5' to 3' order: (i) a 5' AAV ITR; (ii) a promoter; (iii) the polynucleotide according to claim 11; (iv) a polyA; and (v) a 3' AAV ITR;
preferably, wherein:
(a) the rAAV capsid comprises capsid proteins of AAV1, AAV2, AAV5, AAV7, or AAV8, preferably capsid proteins of AAV8; and/or
(b) the promoter is a cytomegalovirus (CMV) promoter, EF-1α promoter, chicken β-actin promoter, GRK1 promoter, or CAG promoter, preferably a CMV promoter; and/or
(c) the polyA is an SV40 late polyA or rabbit globin polyA, preferably an SV40 late polyA; and/or
(d) the 5' AAV ITR and/or the 3' AAV ITR are/is selected from ITRs of AAV1, AAV2, AAV3, AAV4, AAV5, and AAV6; and/or
(e) the recombinant adeno-associated virus is an scAAV, ssAAV, or cceAAV.

15. An expression cassette, comprising in 5' to 3' order: (i) a promoter; (ii) the polynucleotide according to claim 11; and (iii) a poly A;
preferably, wherein:
(a) the promoter is a cytomegalovirus (CMV) promoter, EF-1α promoter, chicken β-actin promoter, GRK1 promoter, or CAG promoter, preferably a CMV promoter; and/or
(b) the polyA is an SV40 late polyA or rabbit globin polyA, preferably an SV40 late polyA; and/or
(c) the expression cassette comprises a 5' AAV ITR and a 3' AAV ITR at the 5' end of the promoter and at the 3' end of the polyA, respectively.

## Amended claims

### Amended claims in accordance with Rule 137(2) EPC.

1. A gene delivery vector, comprising a nucleic acid, wherein the nucleic acid comprises an expression cassette that expresses a human VEGF receptor fusion protein; the expression cassette comprises, in the direction from 5' to 3', a promoter, a nucleic acid encoding the human VEGF receptor fusion protein, and a polyadenylation signal in sequence; the human VEGF receptor fusion protein comprises, from N-terminus to C-terminus, an immunoglobulin-like domain 2 of human VEGFR1, an immunoglobulin-like domain 3 of human VEGFR2, and a hinge-Fc domain of a human immunoglobulin; the hinge-Fc domain of the human immunoglobulin comprises, according to EU numbering, substitutions M252Y, S254T, and T256E.

2. The gene delivery vector according to claim 1, wherein:
(a) the human VEGF receptor fusion protein further comprises an immunoglobulin-like domain 4 of human VEGFR2 between the immunoglobulin-like domain 3 of human VEGFR2 and the hinge-Fc domain of a human immunoglobulin; and/or
(b) the hinge-Fc domain of the human immunoglobulin is a hinge-Fc domain of human IgG1; and/or
(c) the immunoglobulin-like domain 2 of human VEGFR1 comprises or consists of the amino acid sequence set forth in SEQ ID NO. 1 or 6, or an amino acid sequence having at least 80% sequence identity to the amino acid sequence set forth in SEQ ID NO. 1 or 6; and/or
(d) the immunoglobulin-like domain 3 of human VEGFR2 comprises or consists of the amino acid sequence set forth in SEQ ID NO. 2, or an amino acid sequence having at least 80% sequence identity to the amino acid sequence set forth in SEQ ID NO. 2; and/or
(e) the immunoglobulin-like domain 4 of human VEGFR2 comprises or consists of the amino acid sequence set forth in SEQ ID NO. 7, or an amino acid sequence having at least 80% sequence identity to the amino acid sequence set forth in SEQ ID NO. 7; and/or
(f) the hinge-Fc domain of the human immunoglobulin comprises or consists of the amino acid sequence set forth in SEQ ID NO. 3 or 8, or an amino acid sequence having at least 80% sequence identity to the amino acid sequence set forth in SEQ ID NO. 3 or 8.

3. The gene delivery vector according to claim 1, wherein the human VEGF receptor fusion protein comprises or consists of the amino acid sequence set forth in SEQ ID NO. 5 or 9, or an amino acid sequence having at least 80% sequence identity to the amino acid sequence set forth in SEQ ID NO. 5 or 9.

4. The gene delivery vector according to any one of claims 1 to 3, wherein:
(a) the expression cassette comprises: the nucleotide sequence set forth in SEQ ID NO. 10 or 11, or a degenerate sequence of any of SEQ ID NOs. 10 and 11; and/or
(b) the promoter is selected from a cytomegalovirus (CMV) promoter, a Rous sarcoma virus (RSV) promoter, an MMT promoter, an EF-1α promoter, a U6 promoter, a chicken β-actin promoter, a CAG promoter, a CBA promoter, an RPE65 promoter, a VMD2 promoter, an RPGR promoter, an IRBP promoter, an hGRK1 promoter, a CAR promoter, an RHO promoter, a Grm6 promoter, a GRK1 promoter, and a GFAP promoter; and/or
(c) the polyadenylation signal is selected from an SV40 early polyA, an SV40 late polyA, a rabbit globin polyA, a bGH polyA, and an HSV TK polyA.

5. The gene delivery vector according to any one of claims 1 to 4, wherein the gene delivery vector is
(a) a viral vector, preferably a lentivirus, retrovirus, adenovirus, adeno-associated virus, herpesvirus, poxvirus, papovavirus, baculovirus, or papillomavirus, and more preferably an adeno-associated virus or lentivirus; or
(b) a non-viral vector, preferably a plasmid, liposome, nanoparticle, polymer, transposon, exosome, or bacterial vector;

6. The gene delivery vector according to any one of claims 1 to 5, wherein the vector is an adeno-associated virus vector, comprising: (a) a recombinant adeno-associated virus capsid, and (b) the nucleic acid packaged within the recombinant adeno-associated virus capsid;
preferably, wherein
(a) the recombinant adeno-associated virus capsid comprises capsid proteins of an adeno-associated virus selected from the following serotypes: AAV1, AAV2, AAV3, AAV4, AAV5, AAV6, AAV7, AAV8, AAV9, AAV10, AAV11, AAV12, and hybrids thereof, and capsid protein mutants obtained by engineering with the above capsid proteins as backbones; preferably, the recombinant adeno-associated virus capsid comprises capsid proteins of an adeno-associated virus selected from AAV1, AAV2, AAV5, AAV7, AAV8, and hybrids thereof; and/or
(b) the genome of the adeno-associated virus vector is single-stranded or doublestranded and is preferably an scAAV, ssAAV, or cceAAV.

7. A pharmaceutical composition, comprising the gene delivery vector according to any one of claims 1 to 6 and a pharmaceutically acceptable excipient; preferably, the pharmaceutical composition is an injectable formulation.

8. The gene delivery vector according to any one of claims 1 to 6 or the pharmaceutical composition according to claim 7 for use in treating a VEGF-induced disease, wherein preferably the VEGF-induced disease is selected from exudative (wet) age-related macular degeneration (AMD), diabetic retinopathy (DR) combined with macular edema (DME), retinal vein occlusion (RVO) combined with macular edema (ME), central exudative chorioretinopathy, polypoidal choroidal vasculopathy (PCV), choroidal neovascularization secondary to high myopia, and secondary macular choroidal neovascularization (CNV).

9. A recombinant adeno-associated virus, comprising:
(a) an rAAV capsid; and
(b) a nucleic acid packaged within the rAAV capsid, the nucleic acid comprising in 5' to 3' order: (i) a 5' AAV ITR; (ii) a promoter; (iii) a polynucleotide; (iv) a polyA; and (v) a 3' AAV ITR; wherein the polynucleotide encodes a human VEGF receptor fusion protein comprising, from N-terminus to C-terminus, an immunoglobulin-like domain 2 of human VEGFR1, an immunoglobulin-like domain 3 of human VEGFR2, and a hinge-Fc domain of a human immunoglobulin; the hinge-Fc domain of the human immunoglobulin comprises, according to EU numbering, substitutions M252Y, S254T, and T256E;
preferably, wherein:
(a) the human VEGF receptor fusion protein further comprises an immunoglobulin-like domain 4 of human VEGFR2 between the immunoglobulin-like domain 3 of human VEGFR2 and the hinge-Fc domain of a human immunoglobulin; and/or
(b) the hinge-Fc domain of the human immunoglobulin is a hinge-Fc domain of human IgG1; and/or
(c) the immunoglobulin-like domain 2 of human VEGFR1 comprises or consists of the amino acid sequence set forth in SEQ ID NO. 1 or 6, or an amino acid sequence having at least 80% sequence identity to the amino acid sequence set forth in SEQ ID NO. 1 or 6; and/or
(d) the immunoglobulin-like domain 3 of human VEGFR2 comprises or consists of the amino acid sequence set forth in SEQ ID NO. 2, or an amino acid sequence having at least 80% sequence identity to the amino acid sequence set forth in SEQ ID NO. 2; and/or
(e) the immunoglobulin-like domain 4 of human VEGFR2 comprises or consists of the amino acid sequence set forth in SEQ ID NO. 7, or an amino acid sequence having at least 80% sequence identity to the amino acid sequence set forth in SEQ ID NO. 7; and/or
(f) the hinge-Fc domain of the human immunoglobulin comprises or consists of the amino acid sequence set forth in SEQ ID NO. 3 or 8, or an amino acid sequence having at least 80% sequence identity to the amino acid sequence set forth in SEQ ID NO. 3 or 8; and/or
(g) the human VEGF receptor fusion protein comprises or consists of the amino acid sequence set forth in SEQ ID NO. 5 or 9, or an amino acid sequence having at least 80% sequence identity to the amino acid sequence set forth in SEQ ID NO. 5 or 9.the consisting of the amino acid sequence set forth in SEQ ID NO. 5 or 9, or an amino acid sequence having at least 80% sequence identity to the amino acid sequence set forth in SEQ ID NO. 5 or 9; and/or
(h) the polynucleotide has a nucleotide sequence set forth in SEQ ID NO. 10 or 11, or a degenerate sequence of any of SEQ ID NOs. 10 and 11; and/or
(i) the rAAV capsid comprises capsid proteins of AAV1, AAV2, AAV5, AAV7, or AAV8, preferably capsid proteins of AAV8; and/or
(j) the promoter is a cytomegalovirus (CMV) promoter, EF-1α promoter, chicken β-actin promoter, GRK1 promoter, or CAG promoter, preferably a CMV promoter; and/or
(k) the polyA is an SV40 late polyA or rabbit globin polyA, preferably an SV40 late polyA; and/or
(l) the 5' AAV ITR and/or the 3' AAV ITR are/is selected from ITRs of AAV1, AAV2, AAV3, AAV4, AAV5, and AAV6; and/or
(m) the recombinant adeno-associated virus is an scAAV, ssAAV, or cceAAV.

10. An expression cassette, comprising in 5' to 3' order: (i) 5' AAV ITR, (ii) a promoter; (iii) a polynucleotide; (iv) a polyA; and (vi) 3' AAV ITR; wherein the polynucleotide encodes a human VEGF receptor fusion protein comprising, from N-terminus to C-terminus, an immunoglobulin-like domain 2 of human VEGFR1, an immunoglobulin-like domain 3 of human VEGFR2, and a hinge-Fc domain of a human immunoglobulin; the hinge-Fc domain of the human immunoglobulin comprises, according to EU numbering, substitutions M252Y, S254T, and T256E;
preferably, wherein:
(a) the human VEGF receptor fusion protein further comprises an immunoglobulin-like domain 4 of human VEGFR2 between the immunoglobulin-like domain 3 of human VEGFR2 and the hinge-Fc domain of a human immunoglobulin; and/or
(b) the hinge-Fc domain of the human immunoglobulin is a hinge-Fc domain of human IgG1; and/or
(c) the immunoglobulin-like domain 2 of human VEGFR1 comprises or consists of the amino acid sequence set forth in SEQ ID NO. 1 or 6, or an amino acid sequence having at least 80% sequence identity to the amino acid sequence set forth in SEQ ID NO. 1 or 6; and/or
(d) the immunoglobulin-like domain 3 of human VEGFR2 comprises or consists of the amino acid sequence set forth in SEQ ID NO. 2, or an amino acid sequence having at least 80% sequence identity to the amino acid sequence set forth in SEQ ID NO. 2; and/or
(e) the immunoglobulin-like domain 4 of human VEGFR2 comprises or consists of the amino acid sequence set forth in SEQ ID NO. 7, or an amino acid sequence having at least 80% sequence identity to the amino acid sequence set forth in SEQ ID NO. 7; and/or
(f) the hinge-Fc domain of the human immunoglobulin comprises or consists of the amino acid sequence set forth in SEQ ID NO. 3 or 8, or an amino acid sequence having at least 80% sequence identity to the amino acid sequence set forth in SEQ ID NO. 3 or 8; and/or
(g) the human VEGF receptor fusion protein comprises or consists of the amino acid sequence set forth in SEQ ID NO. 5 or 9, or an amino acid sequence having at least 80% sequence identity to the amino acid sequence set forth in SEQ ID NO. 5 or 9.the consisting of the amino acid sequence set forth in SEQ ID NO. 5 or 9, or an amino acid sequence having at least 80% sequence identity to the amino acid sequence set forth in SEQ ID NO. 5 or 9; and/or
(h) the polynucleotide has a nucleotide sequence set forth in SEQ ID NO. 10 or 11, or a degenerate sequence of any of SEQ ID NOs. 10 and 11; and/or
(i) the promoter is a cytomegalovirus (CMV) promoter, EF-1α promoter, chicken β-actin promoter, GRK1 promoter, or CAG promoter, preferably a CMV promoter; and/or
(j) the polyA is an SV40 late polyA or rabbit globin polyA, preferably an SV40 late poly A.
